# EUROPEAN PATENT APPLICATION

(11) **EP 4 026 558 A1**
(43) Date of publication of application: **13.07.2022**
(21) Application number: 20856780.0
(22) Date of filing: 26.08.2020
(51) Int. Cl.: A61K 39/12, A61P 31/12, C07K 14/005, C07K 19/00, C12N 5/10, C12N 1/15, C12N 1/19, C12N 1/21, C12N 15/33, C12N 15/63

(54) **COMPOSITE PROTEIN MONOMER HAVING NON-STRUCTURAL PROTEIN OF VIRUS SUPPORTED THEREON, AGGREGATE OF COMPOSITE PROTEIN MONOMER, AND COMPONENT VACCINE COMPRISING AGGREGATE AS ACTIVE INGREDIENT**

(30) Priority: 27.08.2019 JP 2019154360; 21.05.2020 JP 2020089252
(71) Applicant: Tokyo Institute of Technology, Tokyo 152-8550 (JP); The Kitasato Institute, Tokyo 108-8641 (JP); Denka Company Limited, Tokyo 103-8338 (JP)
(72) Inventor: UENO Takafumi, Tokyo 152-8550 (JP); NGUYEN Dan Que, Tokyo 152-8550 (JP); KATAYAMA Kazuhiko, Tokyo 108-8641 (JP); TODAKA Reiko, Tokyo 108-8641 (JP); HAGA Kei, Tokyo 108-8641 (JP); SAWADA Akihito, Tokyo 108-8641 (JP)
(74) Representative: Henkel & Partner mbB
(86) International application number: PCT/JP2020/032260
(87) International publication number: WO 2021/039873

(57) **Abstract**

The present invention addresses the problem of establishing a means for providing a component vaccine that can elicit immunity to a non-structural protein. The present inventors found that this problem can be solved by providing a component vaccine that comprises, as an active ingredient, an aggregate comprising a trimer and/or a hexamer of a molecular needle on which a non-structural protein of a pathogenic virus is supported.

SEQUENCE LISTING

<110>  Tokyo Institute of Technology
       The Kitasato Institute
       Denka Company Limited

<120>  Composite protein monomer carrying virus nonstructural protein,
       aggregate of said monomer, and component vaccine containing said
       aggregate as active ingredient

<130>  P0060PCT

<150>  JP 2019-154360
<151>  2019-08-27

<150>  JP 2020-089252
<151>  2020-05-21

<160>  30

<170>  PatentIn version 3.5

<210>  1
<211>  16
<212>  PRT
<213>  Artificial Sequence

<220>
<223>  repeating sequence

<400>  1

Leu Glu Gly Ser Asn Thr Ile Phe Val Arg Gly Asp Glu Thr Lys Thr
1               5                   10                  15


<210>  2
<211>  86
<212>  PRT
<213>  Bacteriophage T4

<400>  2

Val Glu Gly Asn Gly Thr Ile Leu Val Lys Gly Asn Val Thr Ile Ile
1               5                   10                  15


Val Glu Gly Asn Ala Asp Ile Thr Val Lys Gly Asp Ala Thr Thr Leu
            20                  25                  30


Val Glu Gly Asn Gln Thr Asn Thr Val Asn Gly Asn Leu Ser Trp Lys
        35                  40                  45


Val Ala Gly Thr Val Asp Trp Asp Val Gly Gly Asp Trp Thr Glu Lys
    50                  55                  60


Met Ala Ser Met Ser Ser Ile Ser Ser Gly Gln Tyr Thr Ile Asp Gly
65                  70                  75                  80

Ser Arg Ile Asp Ile Gly
                85

<210>  3
<211>  125
<212>  PRT
<213>  Bacteriophage P2

<400>  3

Val Glu Gly Asn Gly Thr Ile Leu Val Lys Gly Asn Val Thr Ile Ile
1               5                   10                  15

Val Glu Gly Asn Ala Asp Ile Thr Val Lys Gly Asp Ala Thr Thr Leu
            20                  25                  30

Asp Ala Leu His Ile Arg Phe Pro Asp Gly Ala Val Ile Glu Tyr Glu
        35                  40                  45

Pro Glu Thr Ser Ala Leu Thr Val Ser Gly Ile Lys Thr Ala Ser Val
    50                  55                  60

Thr Ala Ser Gly Ser Val Thr Ala Thr Val Pro Val Val Met Val Lys
65                  70                  75                  80

Ala Ser Thr Arg Val Thr Leu Asp Thr Pro Glu Val Val Cys Thr Asn
                85                  90                  95

Arg Leu Ile Thr Gly Thr Leu Glu Val Gln Lys Gly Gly Thr Met Arg
            100                 105                 110

Gly Asn Ile Glu His Thr Gly Gly Glu Leu Ser Ser Asn
        115                 120                 125

<210>  4
<211>  107
<212>  PRT
<213>  Bacteriophage Mu

<400>  4

Val Glu Gly Asn Gly Thr Ile Leu Val Lys Gly Asn Val Thr Ile Ile
1               5                   10                  15

Val Glu Gly Asn Ala Asp Ile Thr Val Lys Gly Asp Ala Thr Thr Leu
            20                  25                  30

Gly Asp Ala Gly Ile Tyr His His Glu Gly His Arg Ile Arg Leu Thr
        35                  40                  45

Lys Asp Gly Arg Cys Ile Ile Thr Cys Lys Thr Val Glu Val Tyr Ala

50                  55                  60

Asp Glu Ser Met Thr Val Asp Thr Pro Arg Thr Thr Phe Thr Gly Asp
65                  70                  75                  80

Val Glu Ile Gln Lys Gly Leu Gly Val Lys Gly Lys Ser Gln Phe Asp
                85                  90                  95

Ser Asn Ile Thr Ala Pro Asp Ala Ile Ile Asn
            100                 105

<210>  5
<211>  110
<212>  PRT
<213>  Bacteriophage phi 92

<400>  5

Val Glu Gly Asn Gly Thr Ile Leu Val Lys Gly Asn Val Thr Ile Ile
1               5                   10                  15

Val Glu Gly Asn Ala Asp Ile Thr Val Lys Gly Asp Ala Thr Thr Leu
            20                  25                  30

Glu Lys Val Ile Ile Ser Asn Asn Lys Gln Thr Tyr Ala Ser Phe Asp
        35                  40                  45

Pro Asn Gly Asn Ile Ser Val Tyr Asn Thr Gln Gly Met Lys Ile Asp
    50                  55                  60

Met Thr Pro Asn Ser Ile Val Leu Thr Asp Ala Gly Gly Gly Lys Leu
65                  70                  75                  80

Thr Leu Gln Gly Gly Thr Met Thr Tyr Lys Gly Gly Thr Val Asn Leu
                85                  90                  95

Asn Gly Leu Thr Ile Thr Pro Asp Gly Arg Met Thr Asp Ser
            100                 105                 110

<210>  6
<211>  27
<212>  PRT
<213>  Bacteriophage T4

<400>  6

Gly Tyr Ile Pro Glu Ala Pro Arg Asp Gly Gln Ala Tyr Val Arg Lys
1               5                   10                  15

Asp Gly Glu Trp Val Leu Leu Ser Thr Phe Leu
            20                  25

<210>  7
<211>  21
<212>  PRT
<213>  Bacteriophage P2

<400>  7

Gly Lys Val Leu His Thr His Lys His Pro Gly Asp Ser Gly Gly Thr
1               5                   10                  15

Thr Gly Ser Pro Leu
            20

<210>  8
<211>  23
<212>  PRT
<213>  Bacteriophage Mu

<400>  8

Gly Lys Ser Thr Asp Lys His Ile His Arg Gly Asp Ser Gly Gly Thr
1               5                   10                  15

Thr Gly Pro Met Gln Leu Glu
            20

<210>  9
<211>  30
<212>  PRT
<213>  Bacteriophage phi 92

<400>  9

Gly Gly Ile Gly Leu His Thr His Thr His Pro Val Arg Gly Val Glu
1               5                   10                  15

Thr Gly Gly Ser Thr Val Thr Ser Asp Lys Pro Asn Gly Gly
            20                  25                  30

<210>  10
<211>  133
<212>  PRT
<213>  Human norovirus LM14-2 strain

<400>  10

Met Gly Lys Lys Gly Lys Asn Lys Ser Gly Arg Gly Lys Lys His Thr
1               5                   10                  15

Ala Phe Ser Ser Lys Gly Leu Ser Asp Glu Glu Tyr Asp Glu Tyr Lys
            20                  25                  30

Arg Ile Arg Glu Glu Arg Asn Gly Lys Tyr Ser Ile Glu Glu Tyr Leu

35                  40                  45

Gln Asp Arg Asp Arg Tyr Tyr Glu Glu Val Ala Ile Ala Arg Ala Thr
    50                  55                  60

Glu Glu Asp Phe Cys Glu Glu Glu Glu Ala Lys Ile Arg Gln Arg Ile
65                  70                  75                  80

Phe Arg Pro Thr Arg Lys Gln Arg Lys Glu Glu Arg Ala Ser Leu Gly
                85                  90                  95

Leu Val Thr Gly Ser Glu Ile Arg Lys Arg Asn Pro Glu Asp Phe Lys
            100                 105                 110

Pro Lys Gly Lys Leu Trp Ala Asp Asp Asp Arg Ser Val Asp Tyr Asn
        115                 120                 125

Glu Lys Leu Asn Phe
    130

<210>  11
<211>  12774
<212>  DNA
<213>  Artificial Sequence

<220>
<223>  plasmid pHuNoV-LM14-2F

<400>  11
tcgaggccgc ggccgcggtc gactctagag gatccagaca tgataagata cattgatgag      60

tttggacaaa ccacaactag aatgcagtga aaaaaatgct ttatttgtga aatttgtgat     120

gctattgctt tatttgtaac cattataagc tgcaataaac aagttaacaa caacaattgc     180

attcatttta tgtttcaggt tcagggggag gtgtgggagg ttttttaaag caagtaaaac     240

ctctacaaat gtggtatggc tgattatgat cctctagcta gagtcggtgg gcctcggggg     300

cgggtgcggg gtcggcgggg ccgcccgggg tggcttcggt cggagccatg gggtcgtgcg     360

ctcctttcgg tcgggcgctg cgggtcgtgg ggcgggcgtc aggcaccggg cttgcgggtc     420

atgcaccagg tgcgcggtcc ttcgggcacc tcgacgtcgg cggtgacggt gaagccgagc     480

cgctcgtaga aggggaggtt gcggggcgcg gaggtctcca ggaaggcggg caccccggcg     540

cgctcggccg cctccactcc ggggagcacg acggcgctgc ccagaccctt gccctggtgg     600

tcgggcgaga cgccgacggt ggccaggaac cacgcgggct ccttgggccg gtgcggcgcc     660

aggaggcctt ccatctgttg ctgcgcggcc agccgggaac cgctcaactc ggccatgcgc     720

gggccgatct cggcgaacac cgcccccgct tcgacgctct ccggcgtggt ccagaccgcc     780

accgcggcgc cgtcgtccgc gacccacacc ttgccgatgt cgagcccgac gcgcgtgagg     840

aagagttctt gcagctcggt gacccgctcg atgtggcggt ccgggtcgac ggtgtggcgc 900

gtggcggggt agtcggcgaa cgcggcggcg agggtgcgta cggcccgggg gacgtcgtcg 960

cgggtggcga ggcgcaccgt gggcttgtac tcggtcatgg aaggtcgtct ccttgtgagg 1020

ggtcaggggc gtgggtcagg ggatggtggc ggcaccggtc gtggcggccg acctgcaggc 1080

atgcaagcta gctttttgca aaagcctagg cctccaaaaa agcctcctca ctacttctgg 1140

aatagctcag aggccgaggc ggcctcggcc tctgcataaa taaaaaaaat tagtcagcca 1200

tggggcggag aatgggcgga actgggcgga gttaggggcg ggatgggcgg agttaggggc 1260

gggactatgg ttgctgacta attgagatgc atgctttgca tacttctgcc tgctggggag 1320

cctggggact ttccacacct ggttgctgac taattgagat gcatgctttg catacttctg 1380

cctgctgggg agcctgggga ctttccacac cctaactgac acacattcca cagctgcctc 1440

gcgcgtttcg gtgatgacgg tgaaaacctc tgacacatgc agctcccgga gacggtcaca 1500

gcttgtctgt aagcggatgc cgggagcaga caagcccgtc agggcgcgtc agcgggtgtt 1560

ggcgggtgtc ggggcgcagc catgacccag tcacgtagcg atagcggagt gtatactggc 1620

ttaactatgc ggcatcagag cagattgtac tgagagtgca ccatatgcgg tgtgaaatac 1680

cgcacagatg cgtaaggaga aaataccgca tcaggcgctc ttccgcttcc tcgctcactg 1740

actcgctgcg ctcggtcgtt cggctgcggc gagcggtatc agctcactca aaggcggtaa 1800

tacggttatc cacagaatca ggggataacg caggaaagaa catgtgagca aaaggccagc 1860

aaaaggccag gaaccgtaaa aaggccgcgt tgctggcgtt tttccatagg ctccgccccc 1920

ctgacgagca tcacaaaaat cgacgctcaa gtcagaggtg gcgaaacccg acaggactat 1980

aaagatacca ggcgtttccc cctggaagct ccctcgtgcg ctctcctgtt ccgaccctgc 2040

cgcttaccgg atacctgtcc gcctttctcc cttcgggaag cgtggcgctt tctcatagct 2100

cacgctgtag gtatctcagt tcggtgtagg tcgttcgctc caagctgggc tgtgtgcacg 2160

aaccccccgt tcagcccgac cgctgcgcct tatccggtaa ctatcgtctt gagtccaacc 2220

cggtaagaca cgacttatcg ccactggcag cagccactgg taacaggatt agcagagcga 2280

ggtatgtagg cggtgctaca gagttcttga agtggtggcc taactacggc tacactagaa 2340

ggacagtatt tggtatctgc gctctgctga agccagttac cttcggaaaa agagttggta 2400

gctcttgatc cggcaaacaa accaccgctg gtagcggtgg tttttttgtt tgcaagcagc 2460

agattacgcg cagaaaaaaa ggatctcaag aagatccttt gatcttttct acggggtctg 2520

acgctcagtg gaacgaaaac tcacgttaag ggattttggt catgagatta tcaaaaagga 2580

tcttcaccta gatcctttta aattaaaaat gaagttttaa atcaatctaa agtatatatg 2640

agtaaacttg gtctgacagt taccaatgct taatcagtga ggcacctatc tcagcgatct 2700

gtctatttcg ttcatccata gttgcctgac tccccgtcgt gtagataact acgatacggg 2760

agggcttacc atctggcccc agtgctgcaa tgataccgcg agacccacgc tcaccggctc 2820

cagatttatc agcaataaac cagccagccg gaagggccga gcgcagaagt ggtcctgcaa 2880

ctttatccgc ctccatccag tctattaatt gttgccggga agctagagta agtagttcgc 2940

cagttaatag tttgcgcaac gttgttgcca ttgctgcagg catcgtggtg tcacgctcgt 3000

cgtttggtat ggcttcattc agctccggtt cccaacgatc aaggcgagtt acatgatccc 3060

ccatgttgtg caaaaaagcg gttagctcct tcggtcctcc gatcgttgtc agaagtaagt 3120

tggccgcagt gttatcactc atggttatgg cagcactgca taattctctt actgtcatgc 3180

catccgtaag atgcttttct gtgactggtg agtactcaac caagtcattc tgagaatagt 3240

gtatgcggcg accgagttgc tcttgcccgg cgtcaacacg ggataatacc gcgccacata 3300

gcagaacttt aaaagtgctc atcattggaa aacgttcttc ggggcgaaaa ctctcaagga 3360

tcttaccgct gttgagatcc agttcgatgt aacccactcg tgcacccaac tgatcttcag 3420

catcttttac tttcaccagc gtttctgggt gagcaaaaac aggaaggcaa aatgccgcaa 3480

aaaagggaat aagggcgaca cggaaatgtt gaatactcat actcttcctt tttcaatatt 3540

attgaagcat ttatcagggt tattgtctca tgagcggata catatttgaa tgtatttaga 3600

aaaataaaca aataggggtt ccgcgcacat ttccccgaaa agtgccacct gacgtctaag 3660

aaaccattat tatcatgaca ttaacctata aaaataggcg tatcacgagg ccctttcgtc 3720

ttcaagaatt aattcgtgag gctccggtgc ccgtcagtgg gcagagcgca catcgcccac 3780

agtccccgag aagttggggg gaggggtcgg caattgaacc ggtgcctaga gaaggtggcg 3840

cggggtaaac tgggaaagtg atgtcgtgta ctggctccgc ctttttcccg agggtggggg 3900

agaaccgtat ataagtgcag tagtcgccgt gaacgttctt tttcgcaacg ggtttgccgc 3960

cagaacacag gtaagtgccg tgtgtggttc ccgcgggcct ggcctcttta cgggttatgg 4020

cccttgcgtg ccttgaatta cttccacgcc cctggctgca gtacgtgatt cttgatcccg 4080

agcttcgggt tggaagtggg tgggagagtt cgaggccttg cgcttaagga gccccttcgc 4140

ctcgtgcttg agttgaggcc tggcctgggc gctggggccg ccgcgtgcga atctggtggc 4200

accttcgcgc ctgtctcgct gctttcgata agtctctagc catttaaaat ttttgatgac 4260

ctgctgcgac gctttttttc tggcaagata gtcttgtaaa tgcgggccaa gatctgcaca 4320

ctggtatttc ggtttttggg gccgcgggcg gcgacggggc ccgtgcgtcc cagcgcacat 4380

gttcggcgag gcggggcctg cgagcgcggc caccgagaat cggacggggg tagtctcaag 4440

ctggccggcc tgctctggtg cctggcctcg cgccgccgtg tatcgccccg ccctgggcgg 4500

caaggctggc ccggtcggca ccagttgcgt gagcggaaag atggccgctt cccggccctg 4560

ctgcagggag ctcaaaatgg aggacgcggc gctcgggaga gcgggcgggt gagtcaccca 4620

cacaaaggaa aagggccttt ccgtcctcag ccgtcgcttc atgtgactcc acggagtacc 4680

gggcgccgtc caggcacctc gattagttct cgacgagctt ttggagtacg tcgtctttag 4740

gttgggggga ggggttttat gcgatggagt ttccccacac tgagtgggtg gagactgaag 4800

ttaggccagc ttggcacttg atgtaattct ccttggaatt tgcccttttt gagtttggat 4860

cttggttcat tctcaagcct cagacagtgg ttcaaagttt ttttcttcca tttcaggtgt 4920

cgtgaggaat tcaagcttgc ctcgaatcac aagtttgtac aaaaaagcag gcttcgaagg 4980

agatagaacc gtgaatgaag atggcgtcta acgacgcttc cgctgccgct gttgccaaca 5040

gcaacaacga catcgcaaaa tcttcaagtg acggtgtgct ttctaacatg gctgtcactt 5100

ttaagcgggc cctcggggcg cggcctaaac agccgccccc gaaggaaata ccacctagac 5160

ccccgcgacc acccacacca gaattggtca aaaagatccc tcctccccca cccaacgggg 5220

aggatgaact agtggtctct tacagcgcca aagatggcgt ttccggactg cctgagctca 5280

ccactgtcag acaaccggaa gaaaccaaca cggcgttcag tgtcccccca ctcaaccaaa 5340

gggagagcag ggacgccaag gagccactaa ctggaacaat cattgaaatg tgggatggag 5400

aaatctacca ttacggcctg tacgtggaac gaggtcttat acttggtgtg cacaagccac 5460

cggcagccat tagccttgcc aaggtcgagc tagcaccgct ctctttgttc tggagacctg 5520

tatacacccc ccagtatctc atctctccag acactcttag gagattacat ggagagtcat 5580

tcccctacac tgcatttgac aacaattgct acgccttttg ttgttgggta ttagacctaa 5640

acgactcatg gctaagcagg agaatgattc agagaacaac aggcttcttc aggccgtacc 5700

aggattggaa caggaaaccc ctccccacta tggatgattc caaattaaag aaggtagcca 5760

acatattctt gtgcactttg tcttcactat tcaccagacc cattaaggac ataataggga 5820

agttgaaacc tcttaacatc cttaacattc tggctacatg tgattggacc ttcgcaggca 5880

tagtggaatc ctcaatactc ttggcagaac tctttggagt tttctggaca cccccagatg 5940

tgtctgcgat gatcgccccc ttgctaggtg attatgaact gcaaggacct gaggaccttg 6000

cagtggaact ggtcccaata gtaatggggg ggataggttt ggtgctagga tttaccaaag 6060

agaaaatcgg aaagatgcta tcatccgctg catccacttt aagagcttgt aaagaccttg 6120

gtgcatacgg actggaaatc ttaaaattgg tcatgaagtg gttcttccca aagaaagagg 6180

aagcaaatga actggctatg gtgagatcca tcgaggatgc agtactagac ctcgaggcaa 6240

ttgaaaacaa ccacatgacc accctactca aagacaaaga cagcttggca acctacatga 6300

gaacccttga ccttgaggag gagaaagcca gaaaactctc aaccaaatct gcttcacccg 6360

atattgtggg cacaatcaac tctcttctgg caagaatcgc tgctgcacgc tccctagtgc 6420

atcgggcgaa agaagagctc tccagcaggc cgagacctgt cgttgtgatg atatcgggaa 6480

gaccagggat agggaaaact caccttgcca gggagctggc caagaagatc gcggcctccc 6540

tcacagggga ccagcgtgtg ggtcttatcc cgcgcaatgg tgtcgaccac tgggacgcat 6600

acaagggcga aagagttgtc ctatgggacg actatggaat gagcaacccc atccatgatg 6660

ccctcaggtt gcaggagctt gctgacactt gccccctcac gctaaattgt gacagaattg 6720

agaacaaagg gaaagtcttt gacagtgatg ccataattat caccaccaat ctggccaacc 6780

cagcaccact ggattatgtc aactttgaag cgtgctcgag acgcattgat ttcctcgtgt 6840

acgcagaagc ccctgaggtg gagaaggcaa agcgcgactt cccaggtcaa cctgacatgt 6900

ggaagaacgc tttcagtcct gacttctcac acataaaact gtcattggct ccacagggtg 6960

gttttgacaa gaacggcaac accccgcatg gaaaaggggt catgaagacc ctcaccactg 7020

gctccctcat cgcccgagca tcagggttac tccatgagag gctagatgaa tatgaactgc 7080

aaggcccagc cctcaccact ttcaactttg accgcaacaa gatacttgct tttagacagc 7140

ttgctgctga aaacaagtat gggctgatgg acacaatgag agttggaaaa cagctcaagg 7200

atgtcaagac catgtcagac ctcaaacaag cacttaagaa catcgcgatc aagaagtgcc 7260

agatagtgta caatggtggc acctacacac ttgaggctga tggcaagggt agtgtgaaag 7320

ttgacaaagt gcaaagtgcc actgtgcaga ccaacaatga actagccggt gccctacacc 7380

acctaaggtg cgctagaatc agatactatg ttaagtgcgt ccaggaggca ctgtattcca 7440

tcatccaaat cgctggggct gcattcgtca ccacgcgcat cgccaagcgc atgaacatac 7500

agaatctctg gtccaagcca caggtggaag acacagaaga gatggccaac aaagatggtt 7560

gcctaaaacc caaagatgat gaagagtttg tcgtctcatc cgacgacatc aaaactgagg 7620

gcaagaaagg gaagaacaag tccggccgtg gcaagaagca cacagccttt tcaagtaaag 7680

ggctcagtga tgaggagtac gatgagtaca agagaatcag agaagaaagg aatggtaagt 7740

actccataga agagtacctt caggacagag acaggtacta cgaggaggtg gccattgcca 7800

gggcaaccga agaggacttc tgtgaagaag aagaggccaa aatccggcag agaattttta 7860

gaccaacaag gaaacaacgc aaagaagaga gggcctctct cggcttggtc acaggctctg 7920

aaatcaggaa gagaaaccca gaagacttca aacccaaggg aaagctgtgg gctgatgacg 7980

acagaagtgt tgactacaat gagaaactca actttgaggc cccaccaagc atctggtcgc 8040

ggatagtcaa ctttggttca ggctggggct tctgggtctc ccccagtctg tttataacat 8100

caacccatgt cataccccaa ggtgcaaaag agttcttcgg agtccctatc aagcaaatcc 8160

agatacacaa gtcaggtgaa ttctgccggt tgaggttccc aaagccaatc agaactgatg 8220

tgacgggcat gattctagaa gaaggtgcgc ccgaggggac cgtggccaca ctgctcatca 8280

agagaccaac tggagagctc atgcctctgg cagccagaat ggggacccat gcaaccatga 8340

aaattcaggg gcgcacagtt ggagggcaaa tgggtatgct cctgacagga tccaacgcca 8400

agagtatgga cctaggcaca acaccaggcg actgcggctg cccctacatc tacaagaggg 8460

ggaatgacta tgtggtcata ggagtccata cggccgctgc ccgtggagga aacactgtca 8520

tatgtgccac ccaggggagt gagggagaag ccacacttga aggaggtgac agtaaaggga 8580

catactgtgg cgcaccaatc ttgggcccag ggagcgctcc gaatctcagt accaagacta 8640

agttttggag gtcatccaca acaccactcc cacctggcac ctacgaacca gcctacctcg 8700

gtggcaaaga ccctagagtc aaggtggccc cttcattgca acaagttatg agggaccagc 8760

tgaagccatt cacagaaccc agaggcaaac caccaagacc aaatgtgttg gaagctgcca 8820

agaaaaccat catcaatgtc cttgagcaaa cgattgatcc accccaaaaa tggtcatttg 8880

cgcaagcttg cgcatccctt gacaaaacca cctccagcgg ccacccgcac cacatgcgga 8940

aaaacgactg ttggaatggg gagtccttca caggaaaatt ggctgatcaa gcctccaagg 9000

ccaacctaat gtttgaagag ggaaagaaca tgactccagt ctacacaggt gcacttaaag 9060

atgagttggt aaagaccgat aaagtttatg gtaaggtcaa gaagaggctt ctgtggggtt 9120

cagatctggc gaccatgata cggtgcgccc gagcttttgg aggccttatg gatgaactca 9180

aggcacactg tgtcacactt cctgtcagag ttggtatgaa catgaatgag gatggcccca 9240

tcatctttga gaagcactcc agatatagat atcactatga tgctgattat tcccggtggg 9300

actcaacaca acaaagggat gtgctagcag cagcactaga aatcatggtt aagttctctc 9360

cagaaccaca cctggcccag atagttgcag aagacctcct ttcccctagc gtgatggatg 9420

taggtgactt tcaaatatca ataagtgagg gtctcccctc tggggtacct tgtacctccc 9480

agtggaattc catcgcccac tggctcctca ctctgtgtgc actctctgaa gtcacggacc 9540

tgtcccctga tatcattcag gccaactccc ttttctcctt ctatggtgat gatgagattg 9600

taagcacaga cataaagttg gacccagaga agctgacagc aaaactcaag gagtacgggc 9660

tgaaaccaac ccgccccgac aaaactgaag gaccccttgt tatctctgaa gacctggatg 9720

gcctgacatt cctccggaga actgtgaccc gtgatccagc tggctggttt ggaaaattgg 9780

aacaaagttc aattctcagg caaatgtact ggaccagggg tcccaaccat gaagatccat 9840

ttgaaacaat gataccacac tcccaaagac ccatacaatt gatgtccctg ctgggcgagg 9900

ctgcactcca cggcccggca ttctatagca aaattagcaa attagtcatt gcagagttga 9960

aggaaggtgg catggatttt tacgtaccca gacaagagcc aatgttcaga tggatgagat 10020

tctcagatct gagcacgtgg gagggcgatc gcaatctggc tcccagtttt gtgaatgaag 10080

atggcgtcga gtgacgccaa cccatctgat gggtccgcag ccaacctcgt cccagaggtc 10140

aacaatgagg ttatggctct ggagcccgtt gttggtgccg ccattgcggc acctgtagcg 10200

ggccaacaaa atgtaattga cccctggatt agaaataatt ttgtacaagc ccctggtgga 10260

gagtttacag tatcccctag aaacgctcca ggtgaaatac tatggagcgc gcccttgggc 10320

cctgatctaa atccctacct atcccatttg gccagaatgt acaatggtta tgcaggtggt 10380

tttgaagtgc aggtaattct cgcggggaac gcgttcaccg ccgggaaggt catatttgca 10440

gcagtcccac caaattttcc aactgaaggc ttgagcccca gccaggtcac tatgttcccc 10500

catatagtag tagatgttag gcaactagaa cctgtgttga ttcccttacc cgatgttagg 10560

aataatttct atcattacaa tcaatcaaat gaccccacca ttaagttgat agcaatgttg 10620

tatacaccac ttagggctaa taatgctggg gatgatgtct tcacagtctc ttgccgagtt 10680

ctcacgagac catcccccga ttttgatttc atatttctag tgccacccac agttgagtca 10740

agaactaaac cattctctgt cccagtttta actgttgagg agatgaccaa ttcaagattc 10800

cccattcctt tggaaaagtt gttcacgggt cccagcagtg cctttgttgt ccaaccacaa 10860

aacggtaggt gcacgactga tggcgtgctc ctaggcacca cccaactgtc tcctgtcaac 10920

atctgcacct tcagaggaga tgtcacccat atcacaggta gtcgtaacta cacaatgaat 10980

ttggcttctc aaaattggag caattatgac ccaacagaag aaatcccagc ccctctagga 11040

actccagatt ttgtggggaa gattcaaggc gtgctcaccc aaaccacaag gacagatggc 11100

tcaacacgcg gccacaaagc cacagtgtac actgggagcg ccggctttgc tccaaaactg 11160

ggtagagttc aatttgaaac tgacacagac catgattttg aagctaacca aaacacaaag 11220

ttcaccccag ttggtgtcat ccaagatggt agcaccaccc accgaaatga accccaacag 11280

tgggtgctcc caagttactc aggcagaaat actcctaatg tgcatctagc ccccgctgta 11340

gcccccactt ttccgggtga gcaacttctc ttctttagat ccaccatgcc cggatgcagc 11400

gggtacccca acatggattt ggactgtctg ctcccccagg aatgggtgca gtacttctac 11460

caagaggcag ccccagcaca atctgatgtg gctctgctaa gatttgtgaa tccagacaca 11520

ggtagggttt tgtttgagtg taagcttcat aaatcaggct atgttacagt ggctcacact 11580

ggccaacatg atttggttat cccccccaat ggttatttta ggtttgattc ctgggtcaac 11640

cagttttaca cgcttgcccc catgggaaat ggaacggggc gtagacgtgc actataatgg 11700

ctggagcttt ctttgctgga ttggcatctg atgtccttgg ctctggactt ggttccctta 11760

tcaatgctgg ggctggggcc atcaaccaaa aagttgagtt tgaaaataac agaaaattgc 11820

aacaagcatc cttccaattt agcagcaatc tacaacaggc ttcctttcaa catgacaaag 11880

agatgctcca agcacaaatt gaggccacca aaaggctaca acaggaaatg atgaaagtta 11940

agcaggcaat gctcctagag ggtgggttct ctgagacaga tgcagcccgc ggggcaatca 12000

acgcccccat gacaaaaact ttggactgga gcgggacaag gtactgggct cccgatgcta 12060

ggactacaac atacaatgca ggccgctttt ccacccctca accatcgggg gcactgccag 12120

gaagagctaa tcttagggat gctgtccctg ctcggggttc ctccagtaag tcttctaatt 12180

cttctactgc cacttctgtg tactcaaatc aaactacttc aacgagactt ggttctacag 12240

ctggttctgg taccagtgtc tcgagcttcc cgtcaactgc aaggactagg agctgggttg 12300

aggatcaaag taggaatttg tcacctttca tgaggggggc ccacaacata tcgtttgtca 12360

ccccaccatc tagcagatcc tctagccaag gcacagtctc aaccgtgcct aaagagattt 12420

tggactcctg gactggcgct ttcaacacgc gcaggcagcc actcttcgct cacattcgta 12480

agcgggggga gtcacggcgg taatgtgaaa agacaaaatt gattatcttt cttttcttta 12540

gtgtctttta aaaaaaaaaa aaaaaaaaaa aaaaaaaaaa aaaaaaaaaa aaaaaaaaaa 12600

aaaaaaaaaa aaaaaaaaaa aaaaaaaagg gtcggcatgg catctccacc tcctcgcggt 12660

ccgacctggg catccgaagg aggacgcacg tccactcgga tggctaaggg agggcgggga 12720

tccggctgct aacaaagccc gaaaggaccc agctttcttg tacaaagtgg tgat 12774

<210> 12
<211> 7639
<212> DNA
<213> Artificial Sequence

<220>
<223> cDNA part in plasmid pHuNoV-LM14-2F

<400> 12
gtgaatgaag atggcgtcta acgacgcttc cgctgccgct gttgccaaca gcaacaacga 60

catcgcaaaa tcttcaagtg acggtgtgct ttctaacatg gctgtcactt ttaagcgggc 120

cctcggggcg cggcctaaac agccgccccc gaaggaaata ccacctagac ccccgcgacc 180

acccacacca gaattggtca aaaagatccc tcctccccca cccaacgggg aggatgaact 240

agtggtctct tacagcgcca aagatggcgt ttccggactg cctgagctca ccactgtcag 300

acaaccggaa gaaaccaaca cggcgttcag tgtcccccca ctcaaccaaa gggagagcag 360

ggacgccaag gagccactaa ctggaacaat cattgaaatg tgggatggag aaatctacca 420

ttacggcctg tacgtggaac gaggtcttat acttggtgtg cacaagccac cggcagccat 480

tagccttgcc aaggtcgagc tagcaccgct ctctttgttc tggagacctg tatacacccc 540

ccagtatctc atctctccag acactcttag gagattacat ggagagtcat tcccctacac 600

tgcatttgac aacaattgct acgccttttg ttgttgggta ttagacctaa acgactcatg 660

gctaagcagg agaatgattc agagaacaac aggcttcttc aggccgtacc aggattggaa 720

caggaaaccc ctccccacta tggatgattc caaattaaag aaggtagcca acatattctt 780

gtgcactttg tcttcactat tcaccagacc cattaaggac ataataggga agttgaaacc 840

tcttaacatc cttaacattc tggctacatg tgattggacc ttcgcaggca tagtggaatc 900

ctcaatactc ttggcagaac tctttggagt tttctggaca cccccagatg tgtctgcgat 960

gatcgccccc ttgctaggtg attatgaact gcaaggacct gaggaccttg cagtggaact 1020

ggtcccaata gtaatggggg ggataggttt ggtgctagga tttaccaaag agaaaatcgg 1080

aaagatgcta tcatccgctg catccacttt aagagcttgt aaagaccttg gtgcatacgg 1140

actggaaatc ttaaaattgg tcatgaagtg gttcttccca aagaaagagg aagcaaatga 1200

actggctatg gtgagatcca tcgaggatgc agtactagac ctcgaggcaa ttgaaaacaa 1260

ccacatgacc accctactca aagacaaaga cagcttggca acctacatga gaacccttga 1320

ccttgaggag gagaaagcca gaaaactctc aaccaaatct gcttcacccg atattgtggg 1380

cacaatcaac tctcttctgg caagaatcgc tgctgcacgc tccctagtgc atcgggcgaa 1440

agaagagctc tccagcaggc cgagacctgt cgttgtgatg atatcgggaa gaccagggat 1500

agggaaaact caccttgcca gggagctggc caagaagatc gcggcctccc tcacagggga 1560

ccagcgtgtg ggtcttatcc cgcgcaatgg tgtcgaccac tgggacgcat acaagggcga 1620

aagagttgtc ctatgggacg actatggaat gagcaacccc atccatgatg ccctcaggtt 1680

gcaggagctt gctgacactt gccccctcac gctaaattgt gacagaattg agaacaaagg 1740

gaaagtcttt gacagtgatg ccataattat caccaccaat ctggccaacc cagcaccact 1800

ggattatgtc aactttgaag cgtgctcgag acgcattgat ttcctcgtgt acgcagaagc 1860

ccctgaggtg gagaaggcaa agcgcgactt cccaggtcaa cctgacatgt ggaagaacgc 1920

tttcagtcct gacttctcac acataaaact gtcattggct ccacagggtg gttttgacaa 1980

gaacggcaac accccgcatg gaaaaggggt catgaagacc ctcaccactg gctccctcat 2040

cgcccgagca tcagggttac tccatgagag gctagatgaa tatgaactgc aaggcccagc 2100

cctcaccact ttcaactttg accgcaacaa gatacttgct tttagacagc ttgctgctga 2160

aaacaagtat gggctgatgg acacaatgag agttggaaaa cagctcaagg atgtcaagac 2220

catgtcagac ctcaaacaag cacttaagaa catcgcgatc aagaagtgcc agatagtgta 2280

caatggtggc acctacacac ttgaggctga tggcaagggt agtgtgaaag ttgacaaagt 2340

gcaaagtgcc actgtgcaga ccaacaatga actagccggt gccctacacc acctaaggtg 2400

cgctagaatc agatactatg ttaagtgcgt ccaggaggca ctgtattcca tcatccaaat 2460

cgctggggct gcattcgtca ccacgcgcat cgccaagcgc atgaacatac agaatctctg 2520

gtccaagcca caggtggaag acacagaaga gatggccaac aaagatggtt gcctaaaacc 2580

caaagatgat gaagagtttg tcgtctcatc cgacgacatc aaaactgagg gcaagaaagg 2640

gaagaacaag tccggccgtg gcaagaagca cacagccttt tcaagtaaag ggctcagtga 2700

tgaggagtac gatgagtaca agagaatcag agaagaaagg aatggtaagt actccataga 2760

agagtacctt caggacagag acaggtacta cgaggaggtg gccattgcca gggcaaccga 2820

agaggacttc tgtgaagaag aagaggccaa aatccggcag agaattttta gaccaacaag 2880

gaaacaacgc aaagaagaga gggcctctct cggcttggtc acaggctctg aaatcaggaa 2940

gagaaaccca gaagacttca aacccaaggg aaagctgtgg gctgatgacg acagaagtgt 3000

tgactacaat gagaaactca actttgaggc cccaccaagc atctggtcgc ggatagtcaa 3060

ctttggttca ggctggggct tctgggtctc ccccagtctg tttataacat caacccatgt 3120

cataccccaa ggtgcaaaag agttcttcgg agtccctatc aagcaaatcc agatacacaa 3180

gtcaggtgaa ttctgccggt tgaggttccc aaagccaatc agaactgatg tgacgggcat 3240

gattctagaa gaaggtgcgc ccgaggggac cgtggccaca ctgctcatca agagaccaac 3300

tggagagctc atgcctctgg cagccagaat ggggacccat gcaaccatga aaattcaggg 3360

gcgcacagtt ggagggcaaa tgggtatgct cctgacagga tccaacgcca agagtatgga 3420

cctaggcaca acaccaggcg actgcggctg cccctacatc tacaagaggg ggaatgacta 3480

tgtggtcata ggagtccata cggccgctgc ccgtggagga aacactgtca tatgtgccac 3540

ccaggggagt gagggagaag ccacacttga aggaggtgac agtaaaggga catactgtgg 3600

cgcaccaatc ttgggcccag ggagcgctcc gaatctcagt accaagacta agttttggag 3660

gtcatccaca acaccactcc cacctggcac ctacgaacca gcctacctcg gtggcaaaga 3720

ccctagagtc aagggtggcc cttcattgca acaagttatg agggaccagc tgaagccatt 3780

cacagaaccc agaggcaaac caccaagacc aaatgtgttg gaagctgcca agaaaaccat 3840

catcaatgtc cttgagcaaa cgattgatcc accccaaaaa tggtcatttg cgcaagcttg 3900

cgcatccctt gacaaaacca cctccagcgg ccacccgcac cacatgcgga aaaacgactg 3960

ttggaatggg gagtccttca caggaaaatt ggctgatcaa gcctccaagg ccaacctaat 4020

gtttgaagag ggaaagaaca tgactccagt ctacacaggt gcacttaaag atgagttggt 4080

aaagaccgat aaagtttatg gtaaggtcaa gaagaggctt ctgtggggtt cagatctggc 4140

gaccatgata cggtgcgccc gagcttttgg aggccttatg gatgaactca aggcacactg 4200

tgtcacactt cctgtcagag ttggtatgaa catgaatgag gatggcccca tcatctttga 4260

gaagcactcc agatatagat atcactatga tgctgattat tcccggtggg actcaacaca 4320

acaaagggat gtgctagcag cagcactaga aatcatggtt aagttctctc cagaaccaca 4380

cctggcccag atagttgcag aagacctcct ttcccctagc gtgatggatg taggtgactt 4440

tcaaatatca ataagtgagg gtctcccctc tggggtacct tgtacctccc agtggaattc 4500

catcgcccac tggctcctca ctctgtgtgc actctctgaa gtcacggacc tgtcccctga 4560

tatcattcag gccaactccc ttttctcctt ctatggtgat gatgagattg taagcacaga 4620

cataaagttg gacccagaga agctgacagc aaaactcaag gagtacgggc tgaaaccaac 4680

ccgccccgac aaaactgaag gaccccttgt tatctctgaa gacctggatg gcctgacatt 4740

cctccggaga actgtgaccc gtgatccagc tggctggttt ggaaaattgg aacaaagttc 4800

aattctcagg caaatgtact ggaccagggg tcccaaccat gaagatccat ttgaaacaat 4860

gataccacac tcccaaagac ccatacaatt gatgtccctg ctgggcgagg ctgcactcca 4920

cggcccggca ttctatagca aaattagcaa attagtcatt gcagagttga aggaaggtgg 4980

catggatttt tacgtaccca gacaagagcc aatgttcaga tggatgagat tctcagatct 5040

gagcacgtgg gagggcgatc gcaatctggc tcccagtttt gtgaatgaag atggcgtcga 5100

gtgacgccaa cccatctgat gggtccgcag ccaacctcgt cccagaggtc aacaatgagg 5160

ttatggctct ggagcccgtt gttggtgccg ccattgcggc acctgtagcg ggccaacaaa 5220

atgtaattga cccctggatt agaaataatt ttgtacaagc ccctggtgga gagtttacag 5280

tatcccctag aaacgctcca ggtgaaatac tatggagcgc gcccttgggc cctgatctaa 5340

atccctacct atcccatttg gccagaatgt acaatggtta tgcaggtggt tttgaagtgc 5400

aggtaattct cgcggggaac gcgttcaccg ccgggaaggt catatttgca gcagtcccac 5460

caaattttcc aactgaaggc ttgagcccca gccaggtcac tatgttcccc catatagtag 5520

tagatgttag gcaactagaa cctgtgttga ttcccttacc cgatgttagg aataatttct 5580

atcattacaa tcaatcaaat gaccccacca ttaagttgat agcaatgttg tatacaccac 5640

ttagggctaa taatgctggg gatgatgtct tcacagtctc ttgccgagtt ctcacgagac 5700

catcccccga ttttgatttc atatttctag tgccacccac agttgagtca agaactaaac 5760

cattctctgt cccagtttta actgttgagg agatgaccaa ttcaagattc cccattcctt 5820

tggaaaagtt gttcacgggt cccagcagtg cctttgttgt ccaaccacaa aacggtaggt 5880

gcacgactga tggcgtgctc ctaggcacca cccaactgtc tcctgtcaac atctgcacct 5940

tcagaggaga tgtcacccat atcacaggta gtcgtaacta cacaatgaat ttggcttctc 6000

aaaattggag caattatgac ccaacagaag aaatcccagc ccctctagga actccagatt 6060

ttgtggggaa gattcaaggc gtgctcaccc aaaccacaag gacagatggc tcaacacgcg 6120

gccacaaagc cacagtgtac actgggagcg ccggctttgc tccaaaactg ggtagagttc 6180

aatttgaaac tgacacagac catgattttg aagctaacca aaacacaaag ttcaccccag 6240

ttggtgtcat ccaagatggt agcaccaccc accgaaatga accccaacag tgggtgctcc 6300

caagttactc aggcagaaat actcctaatg tgcatctagc ccccgctgta gcccccactt 6360

ttccgggtga gcaacttctc ttctttagat ccaccatgcc cggatgcagc gggtacccca 6420

acatggattt ggactgtctg ctcccccagg aatgggtgca gtacttctac caagaggcag 6480

ccccagcaca atctgatgtg gctctgctaa gatttgtgaa tccagacaca ggtagggttt 6540

tgtttgagtg taagcttcat aaatcaggct atgttacagt ggctcacact ggccaacatg 6600

atttggttat cccccccaat ggttatttta ggtttgattc ctgggtcaac cagttttaca 6660

cgcttgcccc catgggaaat ggaacggggc gtagacgtgc actataatgg ctggagcttt 6720

ctttgctgga ttggcatctg atgtccttgg ctctggactt ggttccctta tcaatgctgg 6780

ggctggggcc atcaaccaaa aagttgagtt tgaaaataac agaaaattgc aacaagcatc 6840

cttccaattt agcagcaatc tacaacaggc ttcctttcaa catgacaaag agatgctcca 6900

agcacaaatt gaggccacca aaaggctaca acaggaaatg atgaaagtta agcaggcaat 6960

gctcctagag ggtgggttct ctgagacaga tgcagcccgc ggggcaatca acgcccccat 7020

gacaaaaact ttggactgga gcgggacaag gtactgggct cccgatgcta ggactacaac 7080

atacaatgca ggccgctttt ccacccctca accatcgggg gcactgccag gaagagctaa 7140

tcttagggat gctgtccctg ctcggggttc ctccagtaag tcttctaatt cttctactgc 7200

cacttctgtg tactcaaatc aaactacttc aacgagactt ggttctacag ctggttctgg 7260

taccagtgtc tcgagcttcc cgtcaactgc aaggactagg agctgggttg aggatcaaag 7320

taggaatttg tcacctttca tgaggggggc ccacaacata tcgtttgtca ccccaccatc 7380

tagcagatcc tctagccaag gcacagtctc aaccgtgcct aaagagattt tggactcctg 7440

gactggcgct ttcaacacgc gcaggcagcc actcttcgct cacattcgta agcgggggga 7500

gtcacgggcg taatgtgaaa agacaaaatt gattatcttt cttttcttta gtgtcttttt 7560

aaaaaaaaaa aaaaaaaaaa aaaaaaaaaa aaaaaaaaaa aaaaaaaaaa aaaaaaaaaa 7620

aaaaaaaaaa aaaaaaaaa 7639

<210> 13
<211> 399
<212> DNA
<213> Human norovirus LM14-2 strain

<400> 13
ggcaagaaag ggaagaacaa gtccggccgt ggcaagaagc acacagcctt ttcaagtaaa 60

gggctcagtg atgaggagta cgatgagtac aagagaatca gagaagaaag gaatggtaag 120

tactccatag aagagtacct tcaggacaga gacaggtact acgaggaggt ggccattgcc 180

agggcaaccg aagaggactt ctgtgaagaa gaagaggcca aaatccggca gagaattttt 240

agaccaacaa ggaaacaacg caaagaagag agggcctctc tcggcttggt cacaggctct 300

gaaatcagga agagaaaccc agaagacttc aaacccaagg gaaagctgtg ggctgatgac 360

gacagaagtg ttgactacaa tgagaaactc aactttgag 399

<210> 14
<211> 9
<212> PRT
<213> Artificial Sequence

<220>

<223>  linker

<400>  14

Ser Asn Ser Ser Ser Val Pro Gly Gly
1               5


<210>  15
<211>  5
<212>  PRT
<213>  Artificial Sequence

<220>
<223>  linker

<400>  15

Gly Gly Gly Gly Ser
1               5


<210>  16
<211>  5
<212>  PRT
<213>  Artificial Sequence

<220>
<223>  linker

<400>  16

Pro Ala Pro Ala Pro
1               5


<210>  17
<211>  8
<212>  PRT
<213>  Artificial Sequence

<220>
<223>  modified sequence

<400>  17

Val Glu His His His His His His
1               5


<210>  18
<211>  34
<212>  DNA
<213>  Artificial Sequence

<220>
<223>  primer

<400>  18
acgccatatg ggcaagaaag ggaagaacaa gtcc                                34


<210>  19

<211>  44
<212>  DNA
<213>  Artificial Sequence

<220>
<223>  primer

<400>  19
gctcgaattc gactcaaagt tgagtttctc attgtagtca acac                        44


<210>  20
<211>  44
<212>  DNA
<213>  Artificial Sequence

<220>
<223>  primer

<400>  20
ccggctccgg ccccactcga gggaagcaat acaatatttg tacg                        44


<210>  21
<211>  32
<212>  DNA
<213>  Artificial Sequence

<220>
<223>  primer

<400>  21
ctcaaagttg agtttctcat tgtagtcaac ac                                     32


<210>  22
<211>  44
<212>  DNA
<213>  Artificial Sequence

<220>
<223>  primer

<400>  22
ggaggcgggg gttcactcga gggaagcaat acaatatttg tacg                        44


<210>  23
<211>  241
<212>  PRT
<213>  respiratory syncytial virus

<400>  23

Met Glu Lys Phe Ala Pro Glu Phe His Gly Glu Asp Ala Asn Asn Arg
1               5                   10                  15


Ala Thr Lys Phe Leu Glu Ser Ile Lys Gly Lys Phe Thr Ser Pro Lys
            20                  25                  30


Asp Pro Lys Lys Lys Asp Ser Ile Ile Ser Val Asn Ser Ile Asp Ile

35                  40                  45

Glu Val Thr Lys Glu Ser Pro Ile Thr Ser Asn Ser Thr Ile Ile Asn
    50                  55                  60

Pro Thr Asn Glu Thr Asp Asp Asn Ala Gly Asn Lys Pro Asn Tyr Gln
65                  70                  75                  80

Arg Lys Pro Leu Val Ser Phe Lys Glu Asp Pro Ile Pro Ser Asp Asn
                85                  90                  95

Pro Phe Ser Lys Leu Tyr Lys Glu Thr Ile Glu Thr Phe Asp Asn Asn
            100                 105                 110

Glu Glu Glu Ser Ser Tyr Ser Tyr Glu Glu Ile Asn Asp Gln Thr Asn
        115                 120                 125

Asp Asn Ile Thr Ala Arg Leu Asp Arg Ile Asp Glu Lys Leu Ser Glu
    130                 135                 140

Ile Leu Gly Met Leu His Thr Leu Val Val Ala Ser Ala Gly Pro Thr
145                 150                 155                 160

Ser Ala Arg Asp Gly Ile Arg Asp Ala Met Val Gly Leu Arg Glu Glu
                165                 170                 175

Met Ile Glu Lys Ile Arg Thr Glu Ala Leu Met Thr Asn Asp Arg Leu
            180                 185                 190

Glu Ala Met Ala Arg Leu Arg Asn Glu Glu Ser Glu Lys Met Ala Lys
        195                 200                 205

Asp Thr Ser Asp Glu Val Ser Leu Asn Pro Thr Ser Glu Lys Leu Asn
    210                 215                 220

Asn Leu Leu Glu Gly Asn Asp Ser Asp Asn Asp Leu Ser Leu Asp Asp
225                 230                 235                 240

Phe

<210>  24
<211>  726
<212>  DNA
<213>  respiratory syncytial virus

<400>  24
atggaaaagt ttgctcctga attccatgga gaagatgcaa acaacagggc tactaaattc        60

ctagaatcaa taaagggcaa attcacatca cctaaagatc ccaagaaaaa agatagtatc 120

atatctgtca actcaataga tatagaagta accaaagaaa gccctataac atcaaattca 180

accattatta acccaacaaa tgagacagat gataatgcag ggaacaagcc caattatcaa 240

agaaaacctc tagtaagttt caaagaagac cctataccaa gtgataatcc cttttcaaaa 300

ctatacaaag aaaccataga gacatttgat aacaatgaag aagaatctag ctattcatat 360

gaagaaataa atgatcagac gaacgataat ataactgcaa gattagatag gattgatgaa 420

aaattaagtg aaatactagg aatgcttcac acattagtag tagcaagtgc aggacctaca 480

tctgctaggg atggtataag agatgccatg gttggtttaa gagaagaaat gatagaaaaa 540

atcagaactg aagcattaat gaccaatgac agattagaag ctatggcaag actcaggaat 600

gaggaaagtg aaaagatggc aaaagacaca tcagatgaag tgtctctcaa tccaacatca 660

gagaaattga acaacctgtt ggaagggaat gatagtgaca atgatctatc acttgatgat 720

ttctga 726

<210> 25
<211> 32
<212> DNA
<213> Artificial Sequence

<220>
<223> primer

<400> 25
ggagatatac atatggaaaa gtttgctcct ga 32

<210> 26
<211> 35
<212> DNA
<213> Artificial Sequence

<220>
<223> primer

<400> 26
tgaacccccg cctccgaaat catcaagtga tagat 35

<210> 27
<211> 15
<212> DNA
<213> Artificial Sequence

<220>
<223> primer

<400> 27
ggaggcgggg gttca 15

<210> 28
<211> 21

<212>  DNA
<213>  Artificial Sequence

<220>
<223>  primer

<400>  28
atgtatatct ccttcttaaa g                                                21


<210>  29
<211>  13
<212>  PRT
<213>  respiratory syncytial virus

<400>  29

Asp Met Pro Ile Thr Asn Asp Gln Lys Lys Leu Met Ser
1               5                   10


<210>  30
<211>  574
<212>  PRT
<213>  respiratory syncytial virus

<400>  30

Met Glu Leu Pro Ile Leu Lys Ala Asn Ala Ile Thr Thr Ile Leu Ala
1               5                   10                  15


Ala Val Thr Phe Cys Phe Ala Ser Ser Gln Asn Ile Thr Glu Glu Phe
            20                  25                  30


Tyr Gln Ser Thr Cys Ser Ala Val Ser Lys Gly Tyr Leu Ser Ala Leu
        35                  40                  45


Arg Thr Gly Trp Tyr Thr Ser Val Ile Thr Ile Glu Leu Ser Asn Ile
    50                  55                  60


Lys Glu Asn Lys Cys Asn Gly Thr Asp Ala Lys Val Lys Leu Ile Asn
65                  70                  75                  80


Gln Glu Leu Asp Lys Tyr Lys Asn Ala Val Thr Glu Leu Gln Leu Leu
                85                  90                  95


Met Gln Ser Thr Thr Ala Ala Asn Asn Arg Ala Arg Arg Glu Leu Pro
            100                 105                 110


Arg Phe Met Asn Tyr Thr Leu Asn Asn Thr Lys Lys Thr Asn Val Thr
        115                 120                 125


Leu Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His His Val Asn Ala Gly Lys Ser Thr Thr Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570

## Description

### Technical Field

The present invention relates to a functional protein and to a vaccine employing the protein. More particularly, the invention relates to a composite protein (monomer) formed of a viral non-structural protein (immunogen) with a molecular needle, to an associated product of the monomer, and to a component vaccine containing the associated product as an active ingredient (infection-protective antigen, hereinafter referred to simply as "protective antigen").

### Background Art

There has been provided a technique called "molecular needle," which has been developed focusing on the excellent function of a bacteriophage to introduce genes into a cell (see Patent Document 1).

Previously, the present inventors invented a composite polypeptide containing a structural protein of norovirus attached to a molecular needle to thereby provide a component vaccine against norovirus. The inventors filed a patent application for the invention (see Patent Document 2).

### Prior Art Documents

### Patent Documents

Patent Document 1: Japanese Patent Application Laid-Open (kokai) No. 2015-163056
Patent Document 2: WO 2018/074558, pamphlet

### Disclosure of the Invention

### Problems to be Solved by the Invention

Generally, vaccines are divided into four types: a live vaccine, an inactivated vaccine, a component vaccine, and a toxoid. The live vaccine employs a pathogenic microorganism (e.g., an attenuated virus) as a protective antigen. The inactivated vaccine employs the entirety of a killed pathogenic microorganism as a protective antigen. The component vaccine employs, as a protective antigen, a protein or a sugar chain which is a part (i.e., a component) of an infectious pathogenic microorganism. The toxoid employs a denatured toxin produced by a pathogen as a protective antigen.

In the norovirus component vaccine disclosed in Patent Document 2, a structural protein of norovirus is attached to a molecular needle. On the basis of the function of the molecular needle to penetrate a cell, the structural protein is introduced into cells, to thereby acquire immunity to the structural protein.

However, the gene encoding a viral structural protein readily undergoes mutation, and, heretofore, a large number of virus mutants have been generated. Thus, it is necessary to design a specific structural protein adapted to a possibly prevailing virus strain, to thereby create a suitable vaccine.

Meanwhile, currently implemented component vaccines work on the basis of humoral immunity to a structural component (i.e., structure) of a pathogen as acquired immunity. Thus, the component vaccines fail to elicit acquired immunity with respect to a group of microbial enzymes (i.e., non-structural protein), which are generated during self-replication of a pathogen in a host. In contrast, the live vaccine *per se* is an attenuated microorganism which is designed to exert no pathogenicity, and forms a non-structural protein for the duplication of the microorganism. Therefore, the live vaccine can induce global acquired immunity (cell-mediated immunity and humoral immunity), targeting even the duplication mechanism of a pathogen, in the vaccinated subject. However, although being attenuated, the live vaccine is provided from a live pathogenic virus, and a certain risk is intrinsically involved therein. In an extremely rare case, an attenuated pathogenic virus is reverted to a highly toxic pathogenic virus via mutation. In another case, even an attenuated pathogenic virus may elicit toxicity in a human subject having reduced immunity.

Thus, generally, vaccination with a live vaccine must be performed more cautiously as compared with the case of a component vaccine.

### Means for Solving the Problems

The present inventors have conducted extensive studies to solve the aforementioned problems, and have found that a "non-structural protein of a pathogenic virus" (hereinafter may also be referred to simply as "non-structural protein") is caused to be attached to the aforementioned molecular needle to obtain a composite protein, and the composite protein is used as an active ingredient of a component vaccine, whereby acquired immunity targeting the duplication mechanism of the microorganism can be induced although it is a component vaccine. When an associated product (trimer and/or hexamer) of the molecular needle to which the non-structural protein is attached is singly used or is used in combination with an associated product of the molecular needle to which a structural protein is attached, as an active ingredient(s) of a component vaccine, a global immune effect (humoral immunity and cell-mediated immunity) comparable to that of a live vaccine can be attained, while the intrinsic safety of the component vaccine is secured. Furthermore, the non-structural protein is a protein related to a function of a pathogenic virus in a host (e.g., proliferation). Thus, the non-structural protein is resistive to amino acid variation so as to maintain the above function, whereby gene mutation of the non-structural protein is drastically inhibited as compared to the case of a structural protein. As a result, achievement of common immunogenicity of different virus variants is highly possible, so long as the variants belong to the same pathogenic virus species. Even in the case of a pathogenic virus species which undergoes mutation in structural protein with considerable frequency, target immunity can be effectively acquired.

The present invention provides the following aspects:
(1) a "composite protein" in which a non-structural protein serving as an immunogen is attached to a molecular needle (hereinafter may be referred to as the composite protein of the present invention);
(2) an "associated product" formed of the composite protein as a monomer (hereinafter may be referred to as the associated product of the present invention); and
(3) a "component vaccine" containing the associated product as an active ingredient (protective antigen) (hereinafter may be referred to as the vaccine (or component vaccine) of the present invention).

The "component vaccine" essentially contains a protective antigen as an active ingredient. The concept "component vaccine" excludes the aforementioned live vaccine, inactivated vaccine, and toxoid. The active ingredient employed in the vaccine of the present invention contains the associated product (containing trimer and/or hexamer) of the present invention serving as a protective antigen.

### (1) Composite protein of the present invention

The composite protein of the present invention is a composite protein represented by the amino acid sequence of the following formula (1) and serves as a precursor of the associated product of the present invention. Notably, in formulas (1) and (2), the symbol "-" connecting the amino acid sequence units denotes a simple molecular bond (substantially a peptide bond) which is used to clearly isolate from one another the amino acid sequences each having a specific meaning (e.g., W, L₁, Xₙ, and Y).

Specifically, the composite protein is represented by

W-L₁-Xₙ-Y ··· (1)

[wherein W represents an amino acid sequence including one or more amino acid sequences of a part or the entirety of a pathogenic virus non-structural protein serving as an immunogen; L₁ represents a first linker sequence having 0 to 100 amino acids; X represents an amino acid sequence represented by SEQ ID NO: 1; Y represents an amino acid sequence of a cell introduction domain; and repetition number n of X is an integer of 1 to 3],
wherein the amino acid sequence of the cell introduction domain Y is represented by the following formula (2) :

Y₁-L₂-Y₂-Y₃ ··· (2)

[wherein Y₁ represents any one amino acid sequence selected from the group consisting of SEQ ID NOs: 2 to 5; Y₂ represents any one amino acid sequence selected from the group consisting of SEQ ID NOs: 6 to 9; L₂ represents a second linker sequence having 0 to 30 amino acids; Y₃ represents an amino acid sequence for modification; and either of Y₂ and Y₃ may be absent] .

The number (n) of repetition of amino acid sequence X in Xₙ is preferably 1, but may be 2 or 3.

In the above formula (1), the amino acid sequence represented by Xₙ, Y₁, or Y₂ includes a modified amino acid sequence thereof obtained by deleting, substituting, or adding one or more amino acid residues from, in, or to the original amino acid sequence. The term "deleting" refers to deletion of any amino acid residue in the amino acid sequence included in formula (1) and represented by any of the SEQ ID NOs. The amino acid residue at the N-terminal side of the deleted amino acid residue and that at the C-terminal side of the deleted amino acid residue are linked via a peptide bond. In the case of deletion of the N-terminal amino acid residue or the C-terminal amino acid residue, no linkage is present. The number of deleted residues is counted as "the number of amino acid deletions." The term "substituting" refers to substitution of any amino acid residue in the amino acid sequence included in formula (1) and represented by any of the SEQ ID NOs, "with another amino acid residue." The new amino acid residue is linked to the amino acid residue at the N-terminal side thereof and at the C-terminal side thereof via a peptide bond. In the case of substitution of the N-terminal side or C-terminal side amino acid residue, the amino acid residue is linked via a peptide bond to another C-terminal side or N-terminal side amino acid residue. The number of substituted residues is counted as "the number of amino acid substitutions." The term "adding" refers to addition of one or more new amino acid residues to one or more peptide bond sites in the amino acid sequence included in formula (1) and represented by any of the SEQ ID NOs, to thereby form a new peptide bond(s). The type and number of the above modifications of amino acid residue may be elucidated by comparison in alignment of the amino acid sequence represented by formula (1) with the resultant amino acid sequence through manpower or employment of appropriate analysis software.

The linker sequence L₁ or L₂, or the modification amino acid sequence Y₃ may have any desired sequence within the range of the number of amino acid residues as defined in formula (1).

Further, the trimer or hexamer (as described below) of the modified composite protein having the modified amino acid sequence preferably has substantially the same immunostimulation activity as that of the trimer or hexamer of the composite protein of formula (1). The expression "substantially the same" refers to such a similarity that no significant difference in immunostimulation activity between the trimer or the hexamer of a composite protein having the non-modified amino acid sequence and that of a composite protein having the modified amino acid sequence is confirmed within a significance level range of 5%, when the immunostimulation activity is determined through an established technique such as the "neutralization test."

In the modified amino acid sequence obtained through deletion, substitution, or addition of one or more amino acid residues from, in, or to the amino acid sequence represented by Xₙ, Y₁, or Y₂ included in formula (1), the number of modifications of amino acid residues in each amino acid sequence is preferably ≤8n, more preferably ≤4n, still more preferably ≤2n in the case of Xₙ; preferably ≤30, more preferably ≤20, still more preferably ≤10 in the case of Y₁; and preferably ≤15, more preferably ≤10, still more preferably ≤5 in the case of Y₂.

W is an amino acid sequence including one or more amino acid sequences of a part or the entirety of a non-structural protein of a pathogenic virus. Proteins encoded by a gene of a pathogenic virus are generally divided into a structural protein and a non-structural protein. The structural protein directly forms the structure of the virus, and examples include capsid protein, envelope protein, and core protein. The non-structural protein is a protein encoded by a gene other than a gene or genes encoding the structural protein(s) of the virus. Generally, the non-structural protein is an enzyme protein involved in survival, proliferation, etc. of a virus in a host, and is expressed after entry of the pathogenic virus into the host. In the expression "a part or the entirety of the non-structural protein," "the entirety" specifically refers to "the entirety of each individual non-structural protein of a virus." For example, in the case where the non-structural protein is P protein of an RS virus as mentioned in the Examples, the aforementioned "the entirety" refers to the entire amino acid sequence of the P protein, and the aforementioned "a part" refers to a part of the amino acid sequence of the P protein. In the case of "a part," no particular limitation is imposed on the constitution of the sequence, so long as "a part of each individual non-structural protein of a virus" exhibits antigenicity of the relevant virus.

No particular limitation is imposed on the type of the pathogenic virus from which the non-structural protein is derived for providing a component of W. The pathogenic virus may be a DNA virus, an RNA virus, or a retrovirus. Examples of the pathogenic virus includes not only a pathogenic virus which causes an infection for which a certain vaccine has already been provided, but also a pathogenic virus which causes an infection for which no vaccine has been provided. Examples of the pathogenic virus which causes an infection for which a certain vaccine has already been provided include poliovirus, measles virus, rubella virus, mumps virus, varicella-zoster virus, yellow fever virus, rotavirus, herpes virus, influenza virus, norovirus, rabies virus, Japanese encephalitis virus, hepatitis viruses (e.g., HAV, HBV, and HCV), human papilloma virus, HIV, and ebolavirus. Examples of the pathogenic virus which causes an infection for which no vaccine has been provided include RS virus (RSV). One characteristic feature of the vaccine of the present invention resides in that the vaccine is effective for a virus which causes an infection for which no vaccine has been provided.

No particular limitation is imposed on the animal which may be infected with the pathogenic virus, and examples of the animal include human and vaccine-needed animals. More specific examples of the animal include farm animals (e.g., cow, horse, sheep, pig, goat, or chicken) and pet animals (e.g., dog and cat).

The non-structural protein is individually and specifically selected according to the type of virus targeted. On the basis of information obtained with respect to known virus non-structural proteins, RNA polymerase, DNA polymerase, reverse transcriptase, protease, VPg, NTPase, DNA binding protein, RNA binding protein, transcription factor, phosphorylated protein, L protein, N protein, or the like may be identified and selected. In the present invention, the concept "non-structural protein" also encompasses a protein whose function has not precisely been revealed and which will potentially serve as a non-structural protein, and a protein which functions as both a structural protein and a non-structural protein.

Examples of the non-structural protein of RS virus include NS-1 protein, NS-2 protein, N protein, P protein, M2-1 protein, and L protein. To the genome RNA in a virus particle, N protein, P protein, M2-1 protein, and L protein are linked. These products function as an RNP (i.e., called nucleocapsid, which is a complex of RNA with another viral protein or a ribonucleoprotein complex). These non-structural proteins forming RNP are also present in RS virus particles. Since the non-structural proteins belong to the enzyme group involved in transcription of messenger RNA and replication of the genome, the proteins are synthesized in a large amount in cells after infection.

### (2) Associated product of the present invention

The associated product of the present invention is formed of the composite protein of the present invention as a monomer. More specifically, the associated product includes a trimer or a hexamer of the composite protein, or a mixture of the trimer and the hexamer. Hereinafter, the trimer or the hexamer of the associated products of the present invention and the mixture of the trimer and the hexamer may be collectively referred to simply as a "trimer and/or hexamer." In other words, the associated product of the present invention contains a trimer or hexamer of the composite protein of the present invention as a monomer. Furthermore, in view of the below-mentioned procedure of producing the associated product of the present invention, the associated product may be defined as an associated product formed through association of molecules of the composite protein of the present invention in an aqueous liquid. In some conceivable cases, the associated product of the present invention may fail to be formed depending on the compatibility between the protein to be attached and the relevant molecular needle. In such a case, the target composite protein molecules are brought into contact with one another in an aqueous liquid, and the resultant product is confirmed through SDS-PAGE or the like, so as to easily evaluate whether the target composite protein forms a trimer or a hexamer thereof, and whether or not the product can be used as an active ingredient of the vaccine of the present invention. The thus-formed associated product of the present invention, *per se,* can exert an effect to penetrate a target cells.

The aforementioned trimer is a trimeric protein formed of the same or different composite proteins of the present invention, as a protein monomer, and the aforementioned hexamer is a hexameric protein formed through association of two molecules of the trimeric protein.

### (3) The vaccine of the present invention

The vaccine of the present invention contains the associated product of the present invention as an active ingredient (protective antigen) and serves as a component vaccine against a target pathogenic virus to be administered in a subcutaneous, intradermal, percutaneous, transmucosal, or intramuscular manner. More specifically, the vaccine of the present invention contains, as an active ingredient, the aforementioned trimer and/or hexamer in which one or more peptides or proteins are included as W, the peptides or proteins including one or more of a part or the entirety of a pathogenic virus non-structural protein, and serves as a component vaccine to be administered in a subcutaneous, intradermal, percutaneous, transmucosal, or intramuscular manner. The vaccine of the present invention may contain, as an active ingredient, two or more trimers and/or hexamers in which the entirety or a part of different non-structural proteins from pathogenic virus strains of the same genotype are included as W. Alternatively, the vaccine of the present invention may contain, as active ingredients, two or more trimers and/or hexamers in which the entirety or a part of different non-structural proteins from pathogenic viruses having different genotypes are included as W.

The vaccine of the present invention may include the associated product of the present invention and, as an additional active ingredient, an associated product (including a trimer and/or hexamer) in which a "structural protein" has been attached to a molecular needle (see Patent Document 2), instead of W (non-structural protein) of the trimer and/or hexamer of the present invention. In addition, the vaccine of the present invention may include, as additional active ingredients, associated products in which structural proteins of pathogenic viruses having different genotypes are included as W, in combination. Meanwhile, the structural protein may have a domain to which a neutralizing antibody is bound, the antibody having viral infection inhibiting ability. In such a case, there may also be employed, in combination, an associated product in which one or more peptide sequences of the relevant epitope or the epitope and its vicinity are included as W.

In the case where the aforementioned associated product employed as the active ingredient is formed of a variety of species, the characteristic of acquired immunity becomes similar to that of a live vaccine. Also, when antigenic proteins originating from different pathogenic microorganisms are employed, immunity similar to that of a mixed vaccine can be attained.

In order to further enhance immunogenicity, the vaccine of the present invention may contain an adjuvant, which is, for example, a B subunit of cholera toxin (corresponding to W) attached to a molecular needle. However, the vaccine of the present invention may be an adjuvant-free (i.e., adjuvant-removed) vaccine. The adjuvant-free vaccine is rather preferred, from the viewpoint of attaining an advantageous feature of the vaccine of the present invention.

When multiple species of the aforementioned trimers and/or hexamers are used, no particular limitation is imposed on the ratio of the trimer and/or hexamer species in the vaccine of the present invention, and the ratio may be freely selected in accordance with the purpose of the vaccine to be used.

### (4) The method for producing the associated product of the present invention

The associated product of the present invention is produced through the following procedure. Specifically, 3 or more molecules of the composite protein of the present invention are brought into contact with one another by the mediation of an aqueous liquid, to thereby associate the composite protein molecules as monomers and to form a mixture of a trimer and a hexamer. If needed, the trimer or the hexamer may be selectively isolated and recovered.

The composite protein of the present invention *per se* is formed by expressing a nucleic acid fragment encoding the target composite protein through a genetic engineering procedure. Alternatively, the composite protein is synthesized through a technique of peptide synthesis. When the composite protein molecules are brought into contact with one another in an aqueous liquid, a trimer and a hexamer of the composite protein are formed in a spontaneous manner. As a result, a mixture containing a trimer and a hexamer of the composite protein is provided. Through selectively isolating and recovering the trimer and the hexamer, the trimer or the hexamer of interest can be separately yielded.

Particularly when the composite protein of the present invention is produced through a genetic engineering procedure, the composite protein is biologically formed through gene expression. Thereafter, in one procedure, cells in which the target composite protein has been formed are collected, and broken or lysed to extract the composite protein, and the target composite protein is isolated through a known isolation method. These steps are performed in an aqueous liquid such as water or a buffer. In the aqueous liquid, association proceeds spontaneously, to thereby yield a mixture of the trimer and the hexamer, which is the associated product of the present invention. Alternatively, when the composite protein of the present invention is produced through total chemical synthesis, or produced by individually synthesizing each part and linking the parts through chemical modification, the product is suspended in an aqueous liquid such as water or a buffer. Even in this case, association proceeds spontaneously, to thereby yield a mixture containing the trimer and the hexamer, which is the associated product of the present invention.

No particular limitation is imposed on the method of isolating and recovering the target trimer and hexamer from the mixture containing the target trimer and hexamer, and a known molecular-weight-basis separation method may be employed. Examples of the isolation method include gel electrophoresis, affinity chromatography, molecular sieve separation (e.g., size exclusion chromatography), and ionexchange chromatography.

Thus, one most preferred mode of the associated product production method of the present invention is a production method including culturing, in a liquid culture medium, a transformant into which a nucleic acid fragment encoding the composite protein of the present invention has been incorporated, to thereby form the corresponding composite protein through gene expression; and obtaining a mixture containing a trimer and a hexamer of the composite protein (as a monomer) via a spontaneous association process; or further selectively isolating and recovering the trimer and the hexamer from the resulting mixture.

The thus-produced associated product of the present invention may be used as the active ingredient of the vaccine of the present invention.

### Effects of the Invention

The present invention can provide the following (1) to (3) :
(1) a composite protein (monomer) in which a virus non-structural protein (immunogen) has been attached to a molecular needle, which can be used as an essential unit of an active ingredient (protective antigen) of a component vaccine for efficiently introducing the virus non-structural protein (immunogen) into target tissue cells through subcutaneous, intradermal, percutaneous, transmucosal, or intramuscular administration;
(2) an associated product of the composite protein which can be used as the active ingredient (protective antigen); and
(3) a component vaccine containing the associated product as an active ingredient.

### Brief Description of the Drawings

[Fig. 1]
   A scheme of forming the associated products of the present invention (trimer and hexamer) from the composite protein of the present invention.
[Fig. 2]
   MALDI-TOF mass spectra of PN-sRL-VPg: (a) a signal of monomer, (b) a signal of trimer, and (c) a signal of hexamer (i.e., trimer-dimer). A rough sketch showing the association state of the composite protein of the present invention (i.e., monomer) is attached to each spectrum chart.
[Fig. 3]
   MALDI-TOF mass spectra of PN-sFL-VPg: (a) a signal of monomer, (b) a signal of trimer, and (c) a signal of hexamer (i.e., trimer-dimer). A rough sketch showing the association state of the composite protein of the present invention (i.e., monomer) is attached to each spectrum chart.
[Fig. 4]
   Schematic structure of an RS virus gene.
[Fig. 5]
   Graphs showing an IgG induction effect by nasal inoculation of a component vaccine employed in Example 2 (3 weeks and 7 weeks after inoculation). The active ingredient of the vaccine is the composite protein associated product of the present invention having P protein (a non-structural protein of RS virus).
[Fig. 6]
   Graphs showing an IgA induction effect by nasal inoculation of a component vaccine employed in Example 2 (3 weeks and 7 weeks after inoculation). The active ingredient of the vaccine is the composite protein associated product of the present invention having P protein (a non-structural protein of RS virus).
[Fig. 7]
   A graph showing an inhibitory effect on infection of the lung with RS virus by nasal inoculation of a component vaccine employed in Example 2. The active ingredient of the vaccine is the composite protein associated product of the present invention having P protein (a non-structural protein of RS virus).
[Fig. 8]
   Schematic structure of a peptide of F protein (a structural protein of RS virus employed in Example 3) corresponding to a domain to which a neutralizing antibody is bound (i.e., neutralization epitope), and schematic structure of a plasmid in which four units of the peptide are quadruple-linked and which expresses the composite protein of the present invention having the linked product as an immunogen.
[Fig. 9]
   Graphs showing an IgG induction effect and an IgA induction effect by nasal inoculation of a vaccine (7 weeks after inoculation). The active ingredient of the vaccine is the composite protein associated product of the present invention in which a quadruple-linked product of the neutralization epitope peptide of F protein is attached as immunogen to a molecular needle.
[Fig. 10]
   A graph showing an inhibitory effect on infection of the lung with RS virus by nasal inoculation of a vaccine. The active ingredient of the vaccine is the composite protein associated product of the present invention in which a quadruple-linked product of the neutralization epitope peptide of F protein is attached as immunogen to a molecular needle.

### Modes for Carrying Out the Invention

### (1) The composite protein of the present invention

The composite protein of the present invention is represented by the amino acid sequence of the following formula (1):

W-L₁-Xₙ-Y ··· (1)

[wherein W represents an amino acid sequence including one or more amino acid sequences of a part or the entirety of a pathogenic virus non-structural protein serving as an immunogen; L₁ represents a first linker sequence having 0 to 100 amino acids; X represents an amino acid sequence represented by SEQ ID NO: 1; Y represents an amino acid sequence of a cell introduction domain; repetition number n of X is an integer of 1 to 3].

In formula (1), a specific example of the immunogen W is an amino acid sequence based on a part or the entirety of the peptide structure of the virus non-structural protein. The wording "based on" refers to the immunogen W including the original peptide structure and also a modified amino acid sequence thereof.

The concept "a part or the entirety of a pathogenic virus non-structural protein" has been described above.

The first linker sequence L₁ is required for appropriately maintaining the distance between the immunogen W and the molecular needle part Y, to thereby suppress steric hindrance. As described above, the number of amino acid residues of L₁ is 0 to 100, preferably 4 to 40. No particular limitation is imposed on the specific amino acid sequence, and examples thereof include (GGGGS)ₘ and (PAPAP)ₘ [m represents the number of repetitions and is preferably an integer of 1 to 5, particularly preferably 1 to 3]. Needless to say, the above examples are non-limitative ones.

X is an amino acid residue represented by SEQ ID NO: 1, and the amino acid sequence Xₙ is a sequence of repeating unit Xs with the number of repetitions n (n is an integer). The mode of repetition is linear repetition. The case of X₂ means "X-X" (wherein symbol "-" denotes a peptide bond). Also, in the repeated sequence Xₙ, the amino acid sequence may be modified in the aforementioned manner. As mentioned above, the number n is an integer of 1 to 3. The number n is preferably 1 but may be 2 or 3. The repeated sequence Xₙ (n = 2 or 3) is employed for consistently maintaining the suitable distance between the molecular needle part Y and the immunogen W according to the dimension and characteristics of the immunogen W.

The cell introduction domain Y is a basement of the molecular needle and is based on the tail needle of a bacteriophage (i.e., cell-penetrating part). The domain Y is a protein represented by the following formula (2):

Y₁-L₂-Y₂-Y₃ ··· (2)

[wherein Y₁ represents an amino acid sequence selected from the group consisting of SEQ ID NOs: 2 to 5; Y₂ represents an amino acid sequence selected from the group consisting of SEQ ID NOs: 6 to 9; L₂ represents a second linker sequence having 0 to 30 amino acids; Y₃ represents an amino acid sequence for modification of interest; and Y₂ or Y₃ may be absent].

In Y₁ of formula (2), the amino acid sequence from the end of the N-terminal side to the 32 amino acid residue (32 Leu) corresponds to the amino acid sequence of a triple helix β-sheet structure of the bacteriophage T4. The N-terminal amino acid of valine (1 Val) may also be leucine (1 Leu). The remaining C-terminal amino acid sequence is an amino acid sequence of the needle protein of the bacteriophage on the C-terminal side. Examples of the amino acid sequence which can be used on the C-terminal side of Y₁ include an amino acid sequence of gp5 of bacteriophage T4, an amino acid sequence of gpV of bacteriophage P2, an amino acid sequence of gp45 of bacteriophage Mu, and an amino acid sequence of gp138 of bacteriophage φ92. More specific examples of Y₁ include the amino acid sequence (SEQ ID NO: 2) which has the amino acid sequence of gp5 of bacteriophage T4 on the C-terminal side, the amino acid sequence (SEQ ID NO: 3) which has the amino acid sequence of gpV of bacteriophage P2 on the C-terminal side, the amino acid sequence (SEQ ID NO: 4) which has the amino acid sequence of gp45 of bacteriophage Mu on the C-terminal side, and the amino acid sequence (SEQ ID NO: 5) which has the amino acid sequence of gp138 of bacteriophage φ92 on the C-terminal side. The nucleotide sequence encoding the amino acid sequence of Y₁ may be selected in accordance with a generally known amino acid-nucleobase relationship.

Y₂ in formula (2) is an amino acid sequence of a domain "foldon" of bacteriophage T4 or an amino acid sequence of a domain "tip" of bacteriophage P2, bacteriophage Mu, or bacteriophage φ92. The foldon or tip is a domain forming the tip portion of the molecular needle structure (i.e., fibritin) of bacteriophage. In formula (2), Y₂ is not necessarily present. However, when the amino acid sequence of the foldon or the tip is included, the efficiency of incorporation of the molecular needle to the cell membrane can be enhanced. Thus, the presence of Y₂ is preferred. The amino acid sequence of the foldon of bacteriophage T4 is represented by SEQ ID NO: 6. The nucleotide sequence encoding the amino acid sequence may be selected in accordance with a generally known amino acid-nucleobase relationship.

The amino acid sequence of the tip of bacteriophage P2 is represented by SEQ ID NO: 7. The nucleotide sequence encoding the amino acid sequence may be selected in accordance with a generally known amino acid-nucleobase relationship. The amino acid sequence of the tip of bacteriophage Mu is represented by SEQ ID NO: 8. The nucleotide sequence encoding the amino acid sequence may be selected in accordance with a generally known amino acid-nucleobase relationship. The amino acid sequence of the tip of bacteriophage φ92 is represented by SEQ ID NO: 9. The nucleotide sequence encoding the amino acid sequence may be selected in accordance with a generally known amino acid-nucleobase relationship.

L₂ serves as a second linker intervening between Y₁ and Y₂. The number of amino acid residues in linker L₂ is 0 to 30, preferably 0 to 5. The number of amino acid residue in the linker being 0 means the absence of second linker L₂.

Y₃ is an amino acid sequence for modification and may be selectively incorporated into Y. The modification amino acid sequence is added in order to purify or protect protein (or for another reason), and examples thereof include tag peptides such as histidine tag, GST tag, and FLAG tag. An appropriate linker sequence may be incorporated into Y₃. Such an additional linker sequence may also be a component of the amino acid sequence of Y₃.

The composite protein of the present invention may be produced through a known method; specifically, a genetic engineering process or chemical synthesis. The entirety of the composite protein of the present invention may be produced in a single process. Alternatively, segments of the composite protein are individually produced, and the produced segments are linked through a chemical modification method. In one linking procedure of polypeptides by the mediation of a linker (e.g., L₁ or L₂), a lysine residue or a cysteine residue of one polypeptide may be linked to that of another polypeptide by the mediation of a linker having a succinimido group or a maleimido group.

In a genetic engineering procedure, a nucleic acid fragment encoding the entirety or a part of the target composite protein of the present invention may be expressed in host cells (e.g., *Escherichia coli,* yeast, insect cells, and animal cells) as a transformant, or in a cell-free expression system such as an *E. coli* extract, a rabbit reticulocyte extract, or a wheat germ extract. As an expression vector in which the nucleic acid fragment is incorporated, a vector corresponding to each expression system can be used. Examples of the expression vector include pET (for expression in *E. coli),* pAUR (for expression in yeast), pIEx-1 (for expression in insect cells), pBApo-CMV (for expression in animal cells), and pF3A (for expression in wheat germ extract).

Regarding chemical synthesis, any known method for chemically synthesizing peptides may be employed. More specifically, the entirety or a part of the composite protein of the present invention may be produced through any established customary method (e.g., liquid phase peptide synthesis or solid phase peptide synthesis). Generally known suitable solid phase peptide synthesis techniques (i.e., chemical synthesis) such as a Boc solid phase method or an Fmoc solid phase method may be employed. Also, as described above, a ligation technique may be employed in accordance with need. Needless to say, each amino acid may be produced through a known method, and a commercial product thereof may be used.

### (2) The associated product of the present invention

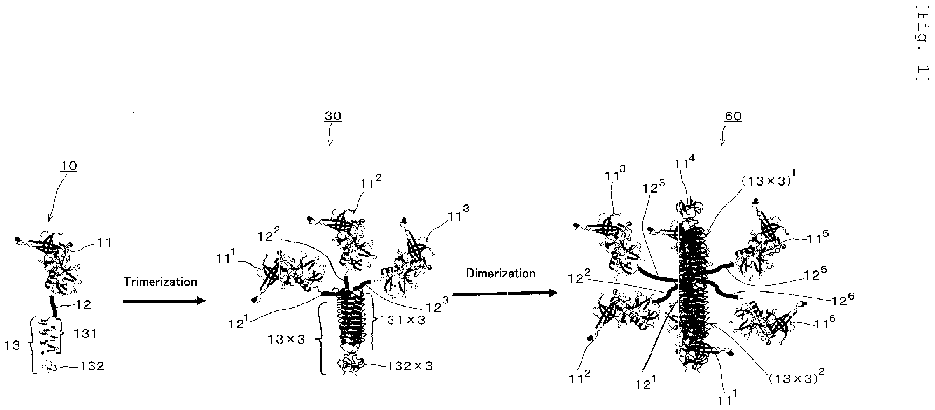

Fig. 1 is a scheme of forming the associated products of the present invention (trimer and hexamer) from the composite protein of the present invention. In Fig. 1, reference numeral 10 denotes the composite protein of the present invention in the form of monomer. Reference numeral 20 denotes the trimer of the present invention, and reference numeral 30 denotes the hexamer of the present invention.

The composite protein 10 of the present invention is formed of a "molecular needle domain 13 corresponding to Y in formula (1)" in which a "base part 131 corresponding to Xₙ and Y₁ in formula (2)" is linked to a "foldon 132 corresponding Y₂ in formula (2)," and an "immunogen 11 corresponding to W in formula (1)," wherein two components are linked together via a "linker 12 corresponding to L₁ in formula (1). A linker or linkers other than linker 12, and the modification sequence corresponding to Y₃ in formula (2) are not illustrated. The composite protein 10 of the present invention *per* se substantially exhibits no function of penetrating the cell membrane of the target cells.

The trimer 30 is an associated product of three molecules of the aforementioned composite protein 10 serving as a monomer via a spontaneous association process. In the trimer 30, 3 units of the molecular needle domain 13 are combined with association via formation of C-terminal-C-terminal bonds, to thereby provide a trimer parallel β-sheet structure, and a helix structure of the β-sheet structure *per se* (i.e., triple helix β-sheet structure), which corresponds to a needle structure. As a result, a molecular needle 13×3 is formed. The molecular needle 13×3 is composed of a basic part 131×3 and a foldon aggregate 132×3. In this way, a "molecular needle" which has a target tissue cell membrane penetration function is formed through trimerization and a self-assembly process. Three linkers (12¹, 12², 12³) originating from the respective monomers, and three immunogen portions (11¹, 11², 11³) linked to the respective linkers exist in the outside area of the molecular needle 13×3.

The hexamer 60 is formed through linking 2 units of the trimer 30, with formation of a bond between the N-terminals of the molecular needle basic parts (13×3)¹ and (13×3)². The hexamer 60 also exhibits a target tissue cell membrane penetration function. Six linkers (12¹, 12², 12³, 12⁵, 126, 12⁴ (12⁴: not illustrated)) originating from the respective trimers, and six immunogen portions (11¹, 11², 11³, 11⁵, 11⁶, 11⁴ (11⁴: not illustrated) linked to the respective linkers exist in the outside area of two molecular needles (13×3)¹ and (13×3)².

Trimerization of the composite protein 10 of the present invention to form the trimer 30, and further dimerization of the trimer 30 to form the hexamer 60 spontaneously proceed in an aqueous liquid. The formed trimer or hexamer exists in a stable state. The stability of the trimer or hexamer is remarkably high. For example, the trimer or hexamer is stable in an aqueous liquid at 100°C, in an aqueous liquid at a pH of 2 to 11, or in an aqueous liquid containing 50 to 70 vol.% of organic solvent. In addition, the trimer or hexamer is excellent in safety. When being isolated from the aqueous liquid and dried, the trimer or hexamer is highly stable and retains an excellent cell membrane penetration function.

As described above, transformation of the composite protein of the present invention to the associated product of interest proceeds spontaneously. Generally, most of the composite protein is hexamized (final form), but some remains as the trimers.

### (3) The vaccine of the present invention

The vaccine of the present invention contains, as an active ingredient, the associated product of the present invention which exhibits an excellent cell penetration function and immunogenicity. Thus, when administered to target tissue cells via subcutaneous administration, intradermal administration, percutaneous administration, mucosal administration, or intramuscular administration, a part or the entirety of the virus non-structural protein serving as the immunogen can be efficiently transferred to the target tissue cells, to thereby attain immunization. As a result, the efficacy and safety of a virus component vaccine via subcutaneous administration, intradermal administration, percutaneous administration, mucosal administration, or intramuscular administration can be enhanced. One demonstrated characteristic feature of the component vaccine of the invention resides in that the vaccine may be used as an adjuvant-free vaccine. No particular limitation is imposed on the target mucosal tissue to which the vaccine is administered, and the target mucosal tissue may be freely selected in accordance with the characters of the target virus (for example, the site affected by the virus). Examples of the target mucosal tissue include the nasal mucous membrane, throat mucous membrane, oral mucous membrane, mucous membrane of bronchus, alimentary canal mucous membrane, and vaginal mucous membrane. In the case of a virus causing respiratory tract inflammation (cold) (e.g., RS virus), suitable targets include the nasal mucous membrane, throat mucous membrane, oral mucous membrane, mucous membrane of bronchus, sublingual mucous membrane, and lung mucous membrane.

As the vaccine for mucosal administration, there will be disclosed a component vaccine containing only a molecular needle to which the aforementioned pathogenic virus structural protein has been attached as an active ingredient.

The vaccine of the present invention is provided as a pharmaceutical composition for subcutaneous administration, intradermal administration, percutaneous administration, mucosal administration, or intramuscular administration, the composition containing the aforementioned associated product of the present invention as an active ingredient (protective antigen). Even in the case of directly administering the associated product of the present invention, the associated product is mixed with and suspended in a buffer or the like upon use, to provide a liquid agent to be subcutaneously, intradermally, percutaneously, mucosally, or intramuscularly administered. Thus, the pharmaceutical composition also encompasses the form of the associated product itself. In the case of the aforementioned mucosal administration, suitable agent forms include spray (e.g., aerosol or spray), capsule, and coating.

The vaccine of the present invention may be prepared in the form of a pharmaceutical composition by blending the associated product of the present invention serving as an essential active ingredient (protective antigen) with an optional molecular needle to which a virus structural protein has been attached and a further optional adjuvant (including the form of the aforementioned molecular needle) and an appropriate pharmaceutical carrier. The pharmaceutical carrier may be selected in accordance with the form of use. Examples of the pharmaceutical carrier which may be used in the invention include a filler, an extender, a binder, a humectant, a disintegrant, a surfactant, an excipient, and a diluent. The form of the composition is generally liquid, but may be a dry product, powder, granule, and the like which are diluted with liquid upon use.

In the vaccine of the present invention, the amount of the associated product of the present invention (in the case where an associated product of a molecular needle to which a structural protein is attached, is also included, the total amount) is not necessarily fixed and may be appropriately chosen. Generally, the associated product of the present invention is suitably used as a liquid formulation containing the product in a dose of 1 to 10 mass% upon administration. The appropriate single dose of administration (inoculation) is about 0.01 µg to about 10 mg for an adult. As needed, the initial inoculation may be appropriately combined with the booster inoculation. The administration (inoculation) may be carried out one or more times.

### Examples

The present invention will next be described in detail by way of example.

The Examples are provided so as to demonstrate the advantageous feature of the associated product of the present invention as the active ingredient of a component vaccine targeted to a virus. In view of difficulty in current production of such a vaccine and usefulness of the production method, RS virus (RSV), also called orthopneumovirus, was selected as an example of the virus.

RS virus is widely identified in the world and can cause lifelong apparent infection of a human subject regardless of age. Particularly in infancy, RS virus is a serious pathogen. Although babies and infants have an antibody to RS virus, which antibody has been transferred from the mother's body, most severe symptoms may be induced in a period of several weeks to several months after birth. In the case of low birth-weight infants, or human subjects having an underlying cardiopulmonary disease or immunodeficiency, the symptoms tend to become more grave. Thus, RS virus has a large impact in clinical settings and on public health.

Currently, there is no authorized RSV vaccine. Previously, some clinical tests were conducted with a formalin-inactivated vaccine. However, the trials failed, since the symptoms of the vaccinated groups were more aggravated than those of the control group. As a pharmaceutical product to cope with RSV infection, Palivizumab, which is a human monoclonal antibody, is only used for prophylactic administeration to a subject in need thereof. Thus, there is demand for an effective vaccine (cited from Web pages of the National Institute of Infectious Disease, Japan).

### [Example 1] Production and identification of the associated product of the present invention

### (a) Introduction

In Example 1, a composite protein falling within the scope of the present invention was prepared through a genetic engineering technique. The composite protein employed, as an attached immunogen, "VPg (viral protein genome-linked)," which serves as a non-structural protein of human norovirus GII.4 (i.e., LM14-2 variant). VPg is a non-structural protein included in open reading frame 1 (ORF1) of the norovirus genome. ORF1 encodes a series of non-structural proteins of nororvirus including N-terminal protein, NTPase (p48), p22 (3A-like), VPg, protease, and RNA-dependent RNA polymerase (RdRp). After completion of total translation, ORF1 is cut by the corresponding protease to provide individual non-structural proteins serving as mature products. Among these mature products, VPg has been found to play an essential role in replication of the norovirus genome via translation from the genomic RNA and the subgenomic RNA. Actually, VPg functions as a cap substitute in mobilization of ribosomes. VPg of LM14-2 variant employed as an immunogen in Example 1 has an amino acid sequence represented by SEQ ID NO: 10 (Notably, Met on the N-terminal originates from start codon ATG). The nucleotide sequence encoding the amino acid sequence may be selected in accordance with a generally known amino acid-nucleobase relationship.

All the reagents employed in Example 1 were purchased from commercial suppliers and used without additional purification. As a gene fragment of HNV-VPg, there was used a gene fragment included in a cDNA fragment (7,640 bases: SEQ ID NO: 12) of human norovirus LM14-2 variant incorporated into plasmid pHuNoV-LM14-2F (1 to 12,550 bases: SEQ ID NO: 11) provided by Katayama, Omura Satoshi Memorial Institute, Kitasato University. VPg is represented by a sequence formed of 372 bases (SEQ ID NO: 13) corresponding to the 2,630th base to the 3,028th base of a cDNA fragment (7,640 bases) of the LM14-2 variant. Start codon ATG was attached to the 5'-terminal of the above sequence, and the product was used in gene expression.

UV-vis spectra were measured by means of SHIMADZU UV-2400PC UV-vis spectrometer. MALDI-TOF mass spectra were measured by means of Bruker ultrafleXtrme. In the MALDI-TOF-MS measurement, each sample was mixed with an equivolume of 70% (v/v) acetonitrile/water containing 0.03% (w/v) sinapic acid and 0.1% (v/v) trifluoroacetic acid. Gel permeation chromatography (GPC) was conducted by means of an HPLC system with a column (Asahipack GF-510HQ, Shodex, Tokyo, Japan).

### (b) Production of plasmid for forming "PN-VPg" via gene expression, and gene expression

### (b)-1: Summary

"PN-VPg" is a composite protein represented by the aforementioned formula (1):

W-L₁-Xₙ-Y ··· (1),

wherein Y represents a cell introduction domain represented by formula (2):

Y₁-L₂-Y₂-Y₃ ··· (2).

In the above formulas, the immunogen W is "LM14-2 variant-VPg" represented by amino acid sequence SEQ ID NO: 10; the first linker L₁ is amino acid sequences SEQ ID NO: 14 (SNSSSVPGG), 15 (GGGGS), or 16 (PAPAP); the repeating unit of the repeating sequence Xₙ is amino acid sequence SEQ ID NO: 1; the number n is 1; the amino acid sequence of the molecular needle base Y₁ is amino acid sequence SEQ ID NO: 2; the second linker L₂ is "SVE"; the amino acid sequence of the foldon Y₂ is amino acid sequence SEQ ID NO: 6; and the amino acid sequence of the modification sequence Y₃ is amino acid sequence SEQ ID NO: 17 (VEHHHHHH).

The plasmid forming PN-VPg via gene expression was constructed from a flexible linker (FL: SNSSSVPGG (SEQ ID NO: 14)) as a template, and based on the plasmid, plasmids were constructed from two shorter linkers; a flexible linker (sFL: GGGGS (SEQ ID NO: 15)) and a rigid linker (sRL: PAPAP (SEQ ID NO: 16)). Through expression of the target protein, an associated product generated spontaneously. The presence of a protein trimer and a protein hexamer was confirmed by analyzing the contents of the associated product. Based on the above, an RS virus component vaccine (i.e., a present target) was produced and tested. As a result, it was demonstrated that the associated product of the present invention employing the virus non-structural protein was remarkably useful for providing a component vaccine.

### (b)-2: Construction of template plasmid by use of flexible linker (FL: SNSSSVPGG (SEQ ID NO: 14))

A VPg segment obtained from LM14-2 plasmid was amplified through polymerase chain reaction (PCR) by use of a gene amplification primer VPg_F (with an *Nde*I restriction enzyme site: ACGCCATATGGGCAAGAAAGGGAAGAACAAGTCC (SEQ ID NO: 18)) and a gene amplification primer VPg_R (with an EcoRI restriction enzyme site: GCTCGAATTCGACTCAAAGTTGAGTTTCTCATTGTAGTCAACAC (SEQ ID NO: 19)). Thereafter, the PCR product was cloned into plasmid pKN1-1 (plasmid for GFP-gp5f expression) (Patent Document 2) digested by NdeI-EcoRI.

The plasmid pKN1-1 was yielded according to the disclosure of Patent Document 2. In the specific procedure, firstly, the gene fragment corresponding to the 461th to the 484th amino acid residues of the wac protein of T4 phage was amplified through PCR from the T4 phage genome, followed by cloning to pUC18, to thereby yield a gene encoding the foldon. Subsequently, the plasmid was cut by restriction enzymes *Eco*RI and SalI, and the product was inserted into a plasmid pET29b (Novagen) treated by EcoRI and *XhoI,* to thereby yield plasmid pMTf1-3. Also, the gene fragment corresponding to the 474th to the 575th amino acid residues of gp5 of the T4 phage was amplified through PCR from the T4 phage genome, followed by cloning to pUC18, to thereby yield a gene encoding gp5. Thereafter, the resultant plasmid was cut by restriction enzymes EcoRI and SalI, and the product was inserted into the aforementioned plasmid pMTf1-3 treated by EcoRI and *XhoI,* to thereby yield plasmid pKA176. Separately, the GFP expression vector provided by Takahasi of Gunma University was cut by restriction enzymes *Nde*I and EcoRI, to thereby yield a gene encoding the GFP. The product was inserted into the aforementioned plasmid pKA176 treated by *Nde*I and EcoRI.

The thus-cloned gene fragment was introduced into competent cells of *E. coli* BL21 (DE3), and the presence of the gene fragment was identified through DNA sequencing. Thus, formation of a plasmid structure including PN and VPg with the interposition of the flexible linker (SNSSSVPGG: SEQ ID NO: 15) "PN-FL-VPg" was confirmed.

### (b)-3: Production of the associated products by use of sFL/sRL linker and identification thereof

Primer combinations for gene amplification were individually used in inverted PCR employing the "PN-FL-VPg" as a template. One primer combination was a gene amplification primer VPgPA-F (with *XhoI* restriction site: CCGGCTCCGGCCCCACTCGAGGGAAGCAATACAATATTTGTACG (SEQ ID NO: 20)) and a gene amplification primer VPgPA-R (CTCAAAGTTGAGTTTCTCATTGTAGTCAACAC (SEQ ID NO: 21)), for interposing a "shorter rigid linker (sRL: PAPAP)" represented by SEQ ID NO: 16 as linker L₁. The other primer combination was a gene amplification primer VPgGS-F (with an *XhoI* restriction site: GGAGGCGGGGGTTCACTCGAGGGAAGCAATACAATATTTGTACG (SEQ ID NO: 22) and a gene amplification primer VPgGS-R (the same as that of the aforementioned VPgPA-R (SEQ ID NO: 21)), for interposing a "shorter flexible linker (sFL: GGGGS)" represented by SEQ ID NO: 15 as linker L₁. As a result, a plasmid structure "PN-sRL-VPg" (wherein L₁ is a shorter rigid linker represented by SEQ ID NO: 16) and a plasmid structure "PN-sFL-VPg" (wherein L₁ is a shorter flexible linker represented by SEQ ID NO: 15) were constructed.

Subsequently, each of the two plasmids was introduced into DH5α competent cells. The thus-obtained vectors were identified through DNA nucleotide sequencing. Thereafter, PN-sRL-VPg and PN-sFL-VPg were formed via gene expression.

Each of the thus-formed "PN-sRL-VPg" and "PN-sFL-VPg" was applied to an Ni affinity column, and the protein contents were eluted with imidazole with a higher concentration gradient of 20 to 500mM and 1mM DTT. Fractions containing PN-VPg were combined, and the volume of the mixture was reduced to 5 mL. The product was dialyzed overnight against a 20mM Tris-HCl buffer (pH: 8.0) at 4°C with a dialysis membrane. The thus-concentrated product was filtered through a 0.2-µm filter, and the filtered liquid was applied to a HiTrap Q column. Then, elution was performed with concentration-graded (0 to 1M) sodium chloride solution. Fractions containing PN-VPg (i.e., shorter linker) were combined, and the combined product was analyzed through Native PAGE and SDS-PAGE.

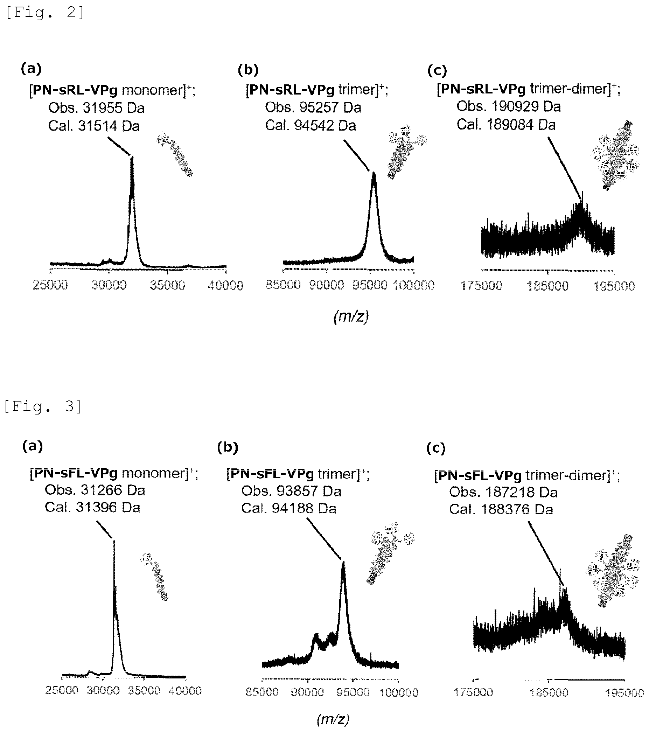

The SDS PAGE analysis showed a band attributable to a monomer having a calculated molecular weight of about 31 kDa, which corresponds to PN-VPg (sFL/sRL linker) (not illustrated). In addition, the MALDI-TOF mass spectra regarding PN-sRL-VPg showed a monomer signal (in Fig. 2(a), m/z (observed): 31,955, m/z (calculated): 31,514), a trimer signal (in Fig. 2(b), m/z (observed): 95,257, m/z (calculated): 94,542), and a hexamer (trimer-dimer) signal (in Fig. 2(c), m/z (observed): 190,929, m/z (calculated): 189,084). Further, the MALDI-TOF mass spectra regarding PN-sFL-VPg showed a PN-sFL-VPg monomer signal (in Fig. 3(a), m/z (observed): 31,266, m/z (calculated): 31,396), a trimer signal (in Fig. 3(b), m/z (observed): 93,857, m/z (calculated): 94,188), and a hexamer (trimer-dimer) signal (in Fig. 3(c), m/z (observed): 187,218, m/z (calculated): 188,376).

From the above experimental results, the associated product of the present invention was found to include a trimer and a hexamer.

### [Example 2] Component vaccine against RS virus

### (a) Introduction

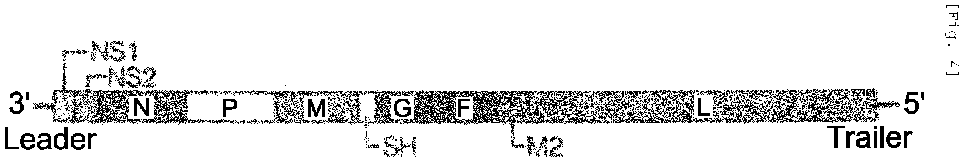

In Example 2, a composite protein falling within the scope of the present invention was prepared through a genetic engineering technique. The composite protein had an immunogen, which is a non-structural protein of a human RS virus Long variant; specifically, "P protein (phosphorylated protein: protein involved in phosphorylation)." P protein is encoded by a template sequence of messenger RNA of P protein in the RS virus genome. Each particle of RS virus has a minus-strand as the genome. As shown in Fig. 4, a complementary chain of messenger RNA of each protein is present at each position. Once cells have been infected with RS virus, messenger RNA of each protein is synthesized by RNP (as described above) using the genomic minus-chain as a template. Each messenger RNA includes an initiation codon and a termination codon. Through translation in cells, corresponding proteins are generated. The virus proteins formed via translation undergo a maturation step and exhibit a virus protein function. P protein forms a tetramer, which binds to L protein and RNP on its the C-terminal side, whereby L protein and RNP are linked by the mediation of the tetramer. On the N-terminal side, P protein serves as a chaperone that can bind free N protein to RNA during synthesis. RNP is surrounded by an envelope originating from a host cell membrane. On the surface of the envelope, two virus glycoproteins; i.e., a receptor binding protein and a fusion protein (F protein), form spikes.

In Example 2, the aforementioned P protein was selected as a non-structural protein component of W. The actually used P protein of RSV Long variant (RSV-P) serving as an immunogen has an amino acid sequence of SEQ ID NO: 23

"MEKFAPEFHGEDANNRATKFLESIKGKFTSPKDPKKKDSIISVNSIDIEVTKESPITSNS
TIINPTNETDDNAGNKPNYQRKPLVSFKEDPIPSDNPFSKLYKETIETFDNNEEESSYSYE
EINDQTNDNITARLDRIDEKLSEILGMLHTLVVASAGPTSARDGIRDAMVGLREEMIEKIR
TEALMTNDRLEAMARLRNEESEKMAKDTSDEVSLNPTSEKLNNLLEGNDSDNDLSLDDF."

The nucleotide sequence encoding the amino acid sequence may be selected in accordance with a generally known amino acid-nucleobase relationship. In Example 2, 
"ATGGAAAAGTTTGCTCCTGAATTCCATGGAGAAGATGCAAACAACAGGGCTACTAAATTC
CTAGAATCAATAAAGGGCAAATTCACATCACCTAAAGATCCCAAGAAAAAGATAGTATCA
TATCTGTCAACTCAATAGATATAGAAGTAACCAAAGAAAGCCCTATAACATCAAATTCAAC
CATTATTAACCCAACAAATGAGACAGATGATAATGCAGGGAACAAGCCCAATTATCAAAGA
AAACCTCTAGTAAGTTTCAAAGAAGACCCTATACCAAGTGATAATCCCTTTTCAAAACTAT
ACAAAGAAACCATAGAGACATTTGATAACAATGAAGAAGAATCTAGCTATTCATATGAAGA
AATAAATGATCAGACGAACGATAATATAACTGCAAGATTAGATAGGATTGATGAAAAATTA
AGTGAAATACTAGGAATGCTTCACACATTAGTAGTAGCAAGTGCAGGACCTACATCTGCTA
GGGATGGTATAAGAGATGCCATGGTTGGTTTAAGAGAAGAAATGATAGAAAAAATCAGAAC
TGAAGCATTAATGACCAATGACAGATTAGAAGCTATGGCAAGACTCAGGAATGAGGAAAGT
GAAAAGATGGCAAAAGACACATCAGATGAAGTGTCTCTCAATCCAACATCAGAGAAATTGA
 ACAACCTGTTGGAAGGGAATGATAGTGACAATGATCTATCACTTGATGATTTC(TGA) (SEQ ID NO: 24)" was employed.

All the reagents employed in Example 2 were purchased from commercial suppliers and used without additional purification. The RSV-P gene fragment was obtained from the genome RNA of RSV-Long variant provided by Sawada, Virus Infection I, Omura Satoshi Memorial Institute, Kitasato University. Specifically, a terminal stop codon (TGA in parenthesis mentioned above) was removed from the P protein gene sequence, and the resultant part was amplified.

### (b) Production and expression of plasmid for forming "PN-P protein" through gene expression

In Example 2, a plasmid structure "PN-FL-P" of interest was constructed through the following procedure. Specifically, the aforementioned RSV-Long variant was subjected to PCR amplification, to amplify the nucleotide sequence (SEQ ID NO: 24) encoding P protein. From the thus-amplified nucleotide sequence (SEQ ID NO: 24) encoding P protein, the terminal stop codon (TGA) was removed, to thereby yield a gene fragment. Using the template plasmid structure "PN-FL-VPg" produced in "Example 1(b)-2" as a template, the gene fragment was substituted with VPg through In-Fusion cloning, to obtain the plasmid structure "PN-FL-P" of interest.

In a more specific procedure, the P domain was amplified by the mediation of In-Fusion RS-P sense 5'-GGAGATATACATATGGAAAAGTTTGCTCCTGA-3' (SEQ ID NO: 25) and In-Fusion RS-P antisense 5'-TGAACCCCCGCCTCCGAAATCATCAAGTGATAGAT-3' (SEQ ID NO: 26), to thereby yield a P domain amplification product into which the underlined portions had been added. Separately, inverted PCR was performed by use of 5'-GGAGGCGGGGGTTCA-3' (SEQ ID NO: 27) and 5'-ATGTATATCTCCTTCTTAAAG-3' (SEQ ID NO: 28) as primers with the template plasmid structure "PN-FL-VPg" as a template, to thereby amplify the entirety of the vector portion excepting the sequence of VPg. Thus, a vector body was provided. These two fragments were linked through In-Fusion cloning, to thereby yield a plasmid structure "PN-FL-P" of interest.

Subsequently, the above plasmid was introduced into DH5α-competent cells. The thus-obtained vector was identified through DNA nucleotide sequencing and then, PN-sFL-P was formed through gene expression.

*E. Coli* BL21 (DE3) having the "PN-sFL-P" plasmid was incubated overnight in an LB medium containing 30 µg/mL Kanamicyn at 37°C. After the OD₆₀₀ of the incubated solution (37°C) had reached 0.8, 1mM isopropyl-β-D-1-thiogalactopyranoside (IPTG) and arabinose were added to the solution. Sixteen (16) to seventeen (17) hours after addition of IPTG and arabinose, the solution was centrifuged at 8,000 rpm for 5 minutes, to thereby recover microorganism pellets, and the recovered matter was incubated at 20°C and a rotation rate of 180 rpm. Subsequently, on ice, the cell pellets were suspended in 100mM Tris-HCl (pH: 8.0) buffer containing 5mM imidazole with one tablet of "cOmplete, EDTA-free," and the cells were lysed through ultrasonication. Cell broken pieces were removed through centrifugation (17,500 rpm for 50 minutes). The supernatant was filtered through a 0.8-µm. filter, and the filtrate was added to an Ni affinity column. The added matter was eluted at 4°C with the same buffer under a linear imidazole concentration gradient of 5mM to 250mM. Thereafter, the PN-FL-P associated product was dialyzed against 20mM Tris-HCl (pH: 8.0) and 0.2M NaCl and further against PBS, and the dialysis product was concentrated through ultrafiltration. Through this process, PN-sFL-P spontaneously yielded an associated product containing a trimer and/or a hexamer thereof. The thus-obtained product was employed in immunoassay as a "PN-sFL-P associated product." As a control, a recombinant P protein (hereinafter abbreviated as P protein) was obtained based on the aforementioned nucleotide sequence encoding the P protein through a customary procedure according to a series of processes from expression to recovery of "PN-sFL-P."

The immunoassay was conducted through nasal immunization by use of 10 µg/mL PBS solution (purified antigen).

### (c) Immunoassay (1) of Example 2 (antibody titer measurement)

Each of the aforementioned PN-sFL-P associated product (PN(+)) and P protein (PN(-)) was nasally inoculated to RS virus-susceptible cotton rats. In all nasal inoculation cases, a vaccine liquid was added dropwise to the nasal cavity, and a purified antigen was caused to be inhaled at 1 mL (10 µg) per rat through the nasal cavity under anesthesia. The nasal inoculation included initial inoculation and booster inoculations three times in total at intervals of 7 days. Blood collection was performed before the initial inoculation, 1 week after the initial inoculation (before the first booster), 2 weeks after the initial inoculation (before the second booster), 3 weeks after the initial inoculation (before the third booster), and 7 weeks after the initial inoculation. Each blood sample was caused to react with RSVP protein, and the IgG titer and the IgA titer to RSVP protein were determined through ELISA.

Eight weeks after the initial inoculation, the final booster was conducted.

In a specific procedure of ELISA, the antigen (RSVP protein) was diluted with PBS(-) to 2 µg/mL. The diluted antigen was added to a 96-well ELISA plate at 50 µL/well and incubated overnight at 4°C. The plate was washed thrice with PBST (0.1% Tween 20/PBS) and then, PBSB (1% BSA/PBS) was added to the plate at 80 µL/well. Incubation was further conducted at room temperature for 2 hours for blocking. Subsequently, a test serum sample was diluted with PBSB, to thereby prepare test samples (for IgG detection: 5-fold dilution series (10-fold to 31,250-fold), and for IgA detection: 3-fold dilution series (10-fold to 2,430-fold). PBSB in the plate was removed, and each test sample was added to the plate at 50 µL/well. Incubation was performed at room temperature for 2 hours, and the plate was washed 5 times with PBST. Then, an HRP substrate solution was added to the plate at 50 µL/well. Incubation was performed at room temperature under light shielding conditions until color development was confirmed. The reaction was stopped by adding 2M sulfuric acid to the plate at 25 µL/well, and absorbance at 490 nm was measured.

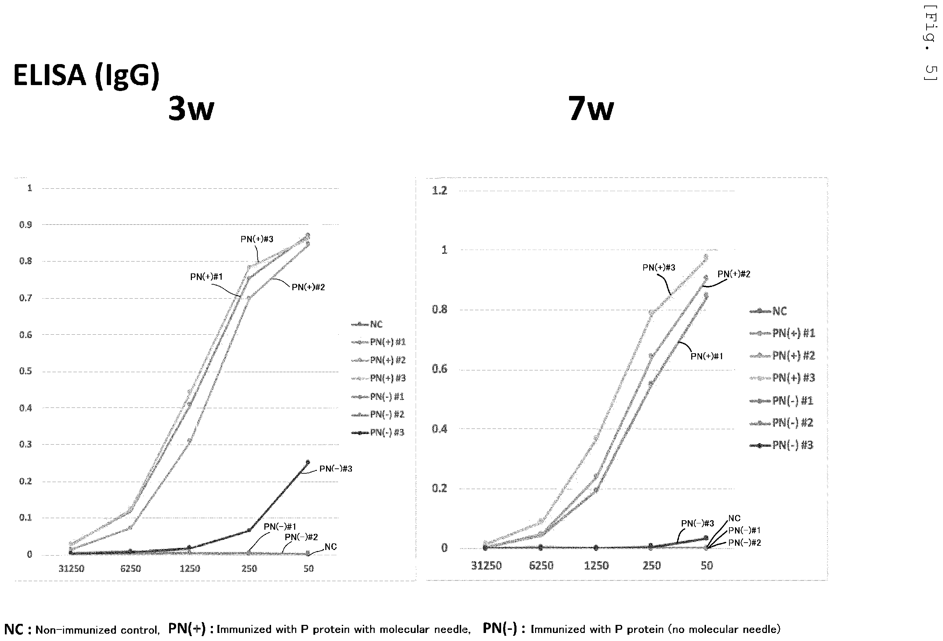

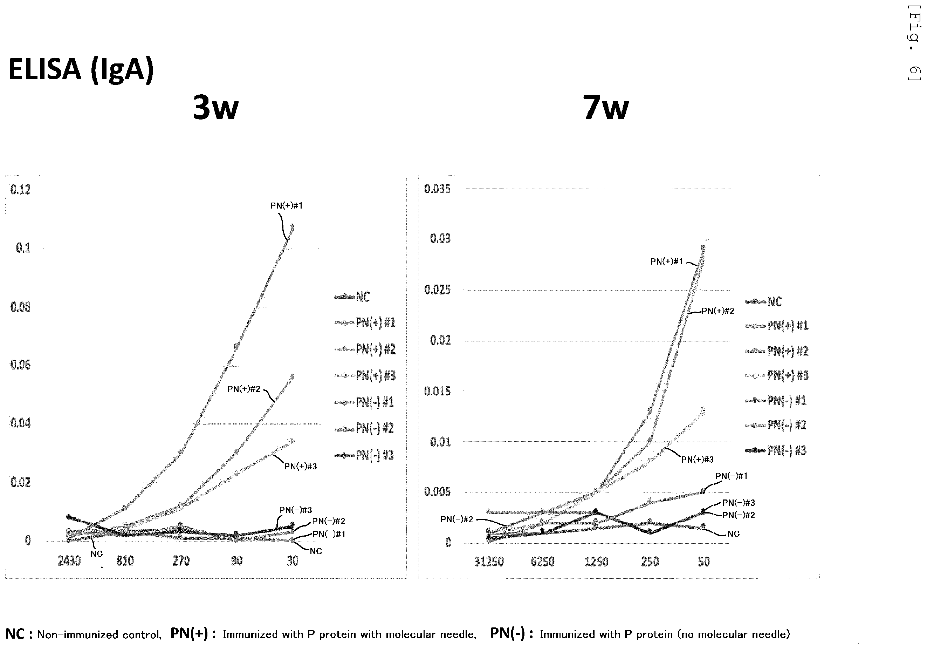

Fig. 5 shows the results of ELISA (IgG), and Fig. 6 shows the results of ELISA (IgA), obtained 3 weeks after the initial inoculation (3W) and 7 weeks after the initial inoculation (7W), respectively. In each graph of Figs. 5 and 6, the vertical axis represents absorbance, and the horizontal axis represents dilution ratio. The graphs show the results of the cotton rats tested under different conditions. As shown in the graphs, when the rats were immunized directly with P protein, no rise in absorbance in response to antibody titer was observed in both cases of IgG and IgA. In contrast, when immunization was conducted with the associated product of the present invention, the antibody titers (i.e., absorbance values) were found to rise remarkably. Furthermore, it was noteworthy that such a remarkable rise in antibody titer was attained by use of no adjuvant. Therefore, the associated product of the present invention can possibly be used as an adjuvant-free vaccine.

An RS virus infection is caused by inhaling the RS virus via droplet infection. That is, the infection mainly is caused via the oral cavity or the nasal cavity. Thus, in Example 2, the associated product was nasally inoculated, whereby the associated product directly penetrated the mucosal cells of the nasal cavity through the cell membranes, resulting in the injection of P protein into the nasal mucosal cells. In the nasal cavity, cell-mediated immunity and humoral immunity were induced by the injected P protein, to thereby elicit protective immunity to RS virus. Thus, when a molecular needle to which a non-structural protein of RS virus was attached was nasally inoculated as an immunogen, topical immunity was found to be induced, to thereby attain an effect of preventing infection with RS virus and an effect of mitigating the infection-related symptoms. Particularly, induction of IgA to P protein, which is a non-structural protein of RS virus, is expected to inhibit the function of P protein through a mechanism including binding to the P protein upon secretion of IgA formed in blood into the mucosal layer through cells.

### (d) Immunoassay (2) of Example 2 (lung inflammatory inhibiting effect)

In the immunoassay (2), RS virus-susceptible cotton rats were immunized with a molecular needle to which an RS virus non-structural protein had been attached, and the rats were tested. Through employment of the rats, the effect of inhibiting lung inflammation caused by RS virus infection was directly investigated.

To each of the RS virus-susceptible cotton rats, the aforementioned PN-sFL-P associated product (PN(+)) or P protein (PN(-)) was nasally inoculated. In all cases of the nasal inoculation, a purified antigen was caused to be administered at 1 mL (10 µg) via inhalation through the nasal cavity under anesthesia. Similar to the case of the aforementioned "Immunoassay (1)," nasal inoculation was performed. Specifically, initial inoculation was performed, and then booster inoculations were conducted three times in total at intervals of 7 days, and the final booster was performed eight weeks after the initial inoculation. Further, one week thereafter, the test rats were infected with RS virus. The dose of RS virus infection was 2×10⁵ PFU/mL, and the infection was conducted in a manner similar to that of the vaccine inoculation (i.e., nasal inoculation).

Four days after the aforementioned infection, the lung tissue was recovered from each tested rat, and the infection amount of RS virus in the lung was determined. Specifically, the lung (i.e., an infection target tissue) was removed from each rat, and suspended in a medium by a homogenizer, to thereby release the RS virus. The amount of infectious virus contained in the lung tissue (50 mg) was determined through plaque assay.

In addition to the tested rats, there were also employed a positive control group (the aforementioned cotton rats infected with RS virus without nasal inoculation in advance) and a negative control group (the aforementioned cotton rats not infected with RS virus and not subjected to nasal inoculation in advance).

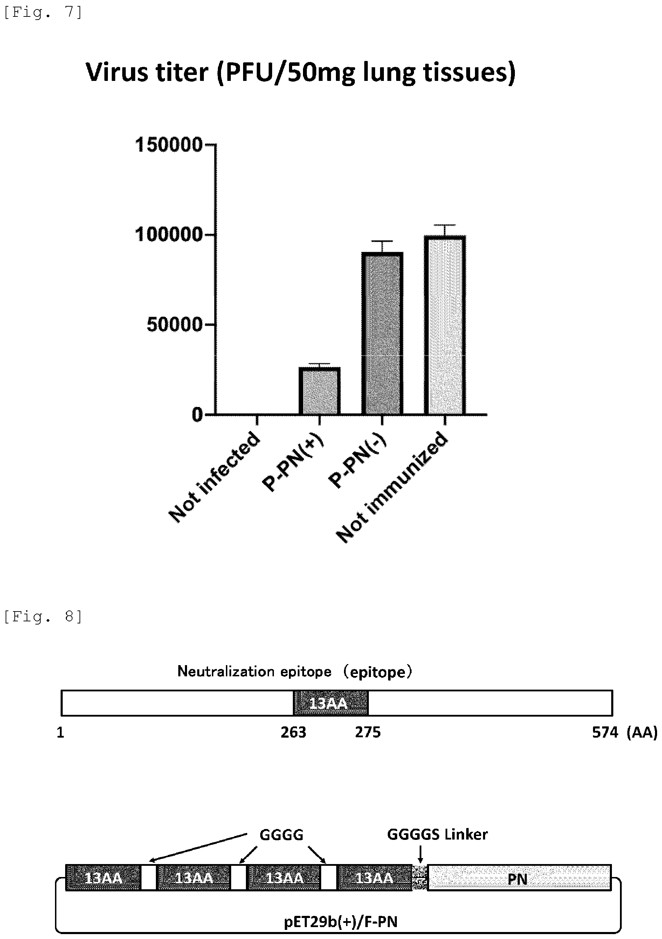

The results are shown in Fig. 7. As shown in Fig. 7, on the horizontal axis, the term "Not infected" represents the negative control (n=1), PN(+) PN-sFL-P associated product (n=3), PN(-) P protein (n=3), and "Not immunized" the positive control (n=3). The vertical axis represents the RS virus titer (PFU/50 mg in lung tissue) of the recovered lung tissue. As is clear from Fig. 7, RS virus infection of rats to which the PN-sFL-P associated product had been administered was found to be inhibited at a ratio of 1/2 or more with a significance of (P<0.0001), as compared with the case of RS virus-infected rats (the positive control).

Based on the above results, the vaccine of the present invention was found to exhibit substantial virus infection inhibitory effect through, in particular, topical immunity induction such as mucosal membrane immunity induction or cell-mediated immunity induction.

### [Example 3] In case where RS virus structural protein is attached

As described above, the RNP of RS virus (RSV) is surrounded by an envelope originating from a host cell membrane. On the surface of the envelope, two virus glycoproteins (structural proteins); i.e., a receptor binding protein and a fusion protein (F protein), form a spike. In Example 3, four units of a peptide formed of 13 amino acids (SEQ ID NO: 29: DMPITNDQKKLMS) forming the neutralization epitope domain of the F protein (as a structural protein) were linked in line, to thereby form a (quadruple) fusion protein. The resultant protein was attached as an immunogen W to a molecular needle, to thereby yield an associated product. The associated product was tested in a manner similar to that of the immunoassay performed in Example 2.

The F protein of RS virus (RSV) is formed of 574 amino acids (SEQ ID NO: 30:

MELPILKANAITTILAAVTFCFASSQNITEEFYQSTCSAVSKGYLSALRTGWYTSVITIEL

SNIKENKCNGTDAKVKLINQELDKYKNAVTELQLLMQSTTAANNRARRELPRFMNYTLNNT

KKTNVTLSKKRKRRFLGFLLGVGSAIASGIAVSKVLHLEGEVNKIKSALLSTNKAVVSLSN

GVSVLTSKVLDLKNYIDKQLLPIVNKQSCRISNIETVIEFQQKNNRLLEITREFSVNAGVT

TPVSTYMLTNSELLSLINDMPITNDQKKLMSNNVQIVRQQSYSIMSIIKEEVLAYVVQLPL

YGVIDTPCWKLHTSPLCTTNTKEGSNICLTRTDRGWYCDNAGSVSFFPQAETCKVQSNRVF

CDTMNSLTLPSEVNLCNVDIFNPKYDCKIMTSKTDVSSSVITSLGAIVSCYGKTKCTASNK

NRGIIKTFSNGCDYVSNKGVDTVSVGNTLYYVNKQEGKSLYVKGEPIINFYDPLVFPSDEF

DASISQVNEKINQSLAFIRKSDELLHHVNAGKSTTNIMITTIIIVIIVILLSLIAVGLLLY

CKARSTPVTLSKDQLSGINNIAFSN: GenBank accession # AY911262).

The domain ranging from the 263th amino acid to the 275th amino acid serves as an epitope domain recognized by a neutralizing antibody. A gene encoding a (quadruple) fusion protein in which four units of the epitope domain of F protein were linked in line by the mediation of (GGGG) was created. Then, a plasmid (pET29b(+)/F-PN) which was designed to fuse with PN by the mediation of a linker (GGGGS: SEQ ID NO: 15) was created. *E. coli* (BL21 DE3) cells were transformed with the plasmid (Fig 8), and expression was induced by IPTG. The expression product was recovered as a soluble protein. The aforementioned associated product of the quadruple fusion protein (Fnep×4-PN associated product (PN(+)) was purified with TAIRON, and the immunoassay of Example 3 was conducted.

### (a) Immunoassay (1) of Example 3 (antibody titer measurement)

Each of the Fnep×4-PN associated product (PN(+)) and the quadruple fusion protein of the neutralization epitope of F protein (PN(-)) was nasally inoculated to RS virus-susceptible cotton rats. In all nasal inoculation cases, a vaccine liquid was added dropwise to the nasal cavity, and the vaccine was caused to be inhaled. Specifically, a purified antigen was caused to be inhaled at 1 mL (20 µg) per rat through the nasal cavity under anesthesia. The nasal inoculation included initial inoculation, and then booster inoculations conducted three times in total at intervals of 7 days. Blood collection was performed before the initial inoculation, 1 week after the initial inoculation (before the first booster), 2 weeks after the initial inoculation (before the second booster), 3 weeks after the initial inoculation (before the third booster), and 7 weeks after the initial inoculation. Each blood sample was caused to react with F protein, and the IgG titer and the IgA titer to F protein were determined through ELISA.

Nine weeks after the initial inoculation, the final booster was conducted.

ELISA was performed in a manner similar to that of Example 2. However, as described above, F protein was used as an antigen.

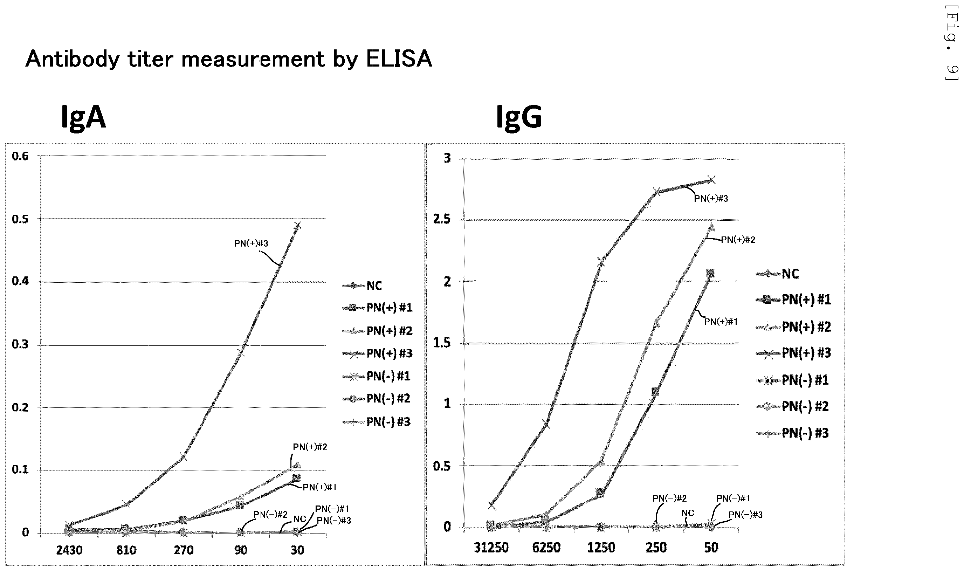

The results (3 weeks after) are shown in Fig. 9 (in terms of IgA and IgG). In each graph of Fig 9, the vertical axis represents absorbance, and the horizontal axis represents dilution ratio. The graphs show the results of the cotton rats tested under different conditions. As shown in the graphs, when the rats were immunized directly with the quadruple fusion protein of the neutralization epitope of F protein, no rise in absorbance in response to antibody titer was observed in both cases of IgG and IgA. In contrast, when immunization was conducted with the Fnep×4-PN associated product, the antibody titers (i.e., absorbance values) were found to rise. The rise in antibody titer was remarkable for IgG, but was observed with variation for IgA.

### (b) Immunoassay (2) of Example 3 (lung inflammatory inhibiting effect)

In immunoassay (2), RS virus-susceptible cotton rats were immunized with a molecular needle to which the quadruple fusion protein of the neutralization epitope of F protein (serving as an RS virus non-structural protein) was attached, and the rats were tested. Through employment of the rats, the effect of inhibiting lung inflammation caused by RS virus infection was directly investigated.

Each of the Fnep×4-PN associated product (PN(+)) and the quadruple fusion protein of the neutralization epitope of F protein (PN(-)) was nasally inoculated to RS virus-susceptible cotton rats. In all nasal inoculation cases, a purified antigen was caused to be inhaled at 1 mL (20 µg) per rat through the nasal cavity under anesthesia. The nasal inoculation was conducted in a manner similar to that of "Immunoassay (1)." The nasal inoculation included initial inoculation and then booster inoculations three times in total at intervals of 7 days. Seven weeks after the initial inoculation, the final booster was conducted. Two weeks after the final booster, the test rats were infected with RS virus. The infection dose of the RS virus was 2×10⁵ PFU/mL, and the infection was conducted in a manner similar to that of the vaccine inoculation (i.e., nasal inoculation).

Four days after the aforementioned infection, the lung tissue was recovered from each tested rat, and the infection amount of RS virus in the lung was determined. Specifically, the lung (i.e., an infection target tissue) was removed from each rat and suspended in a medium by means of a homogenizer, to thereby release the RS virus. The amount of the infectious virus in the lung tissue (50 mg) was determined through plaque assay.

In addition to the test rats, there were also employed a positive control group (the aforementioned cotton rats infected with RS virus without nasal inoculation in advance) and a negative control group (the aforementioned cotton rats not infected with RS virus and not subjected to nasal inoculation in advance).

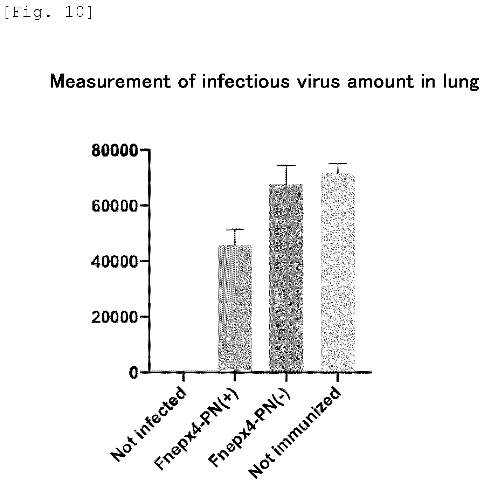

The results are shown in Fig. 10. As shown in Fig. 10, on the horizontal axis, the term "Not infected" represents the negative control (n=1), PN(+) Fnep×4PN associated product (n=3), PN(-) the quadruple fusion protein of the neutralization epitope of F protein (n=3), and "Not immunized" the positive control (n=3). The vertical axis represents the RS virus titer (PFU/50 mg of lung tissue) of the recovered lung tissue. As is clear from Fig. 10, the rats to which the Fnep×4PN associated product had been administrated exhibited a reduced RS virus infection of about 36% with a significance of P<0.0001, as compared with RS virus-infected rats (the positive control).

As is clear from the test results, the needle vaccine containing the associated product having the quadruple fusion protein of the neutralization epitope of F protein (i.e., the structural protein of RS virus) of Example 3 was found to induce topical immunity such as mucosal immunity or cell-mediated immunity. However, the thus-attained effect was found to be weaker than that of the vaccine of the present invention employing a non-structural protein. This indicates that the virus-neutralizing effect of the secretory IgA (sIgA) (i.e., an antibody which was induced through immunization by the associated product having the quadruple fusion protein of the neutralization epitope of F protein, and secreted to the mucin layer of the mucosal epithelium) alone has a limited ability to inhibit virus infection and proliferation. In contrast, the vaccine of the present invention (i.e., the molecular needle employing P protein (a non-structural protein)) was found to more effectively inhibit RS virus infection and proliferation on the basis of cell-mediated immunity induced through immunization with the vaccine of the present invention. In addition, it is considered that the following phenomenon appears as a further effect of the vaccine of the present invention: When IgA molecules synthesized by B cells in blood are taken up into mucosal epithelial cells from the basement membrane side and secreted to the mucosal surface, production of a P-polymerase complex formed between P protein and the polymerase of RS virus is inhibited during passage through the cells.

Further, a component vaccine containing, for example, the associated product of the present invention of Example 2 which has P protein (i.e., a non-structural protein of RS virus), and an associated product of a molecular needle having a structural protein, such as the associated product of Example 3 which has the quadruple fusion protein of the neutralization epitope of F protein (i.e., a structural protein of RS virus), in combination as active ingredients is expected to have a synergistic effect due to the combination.

## Claims

1. A composite protein represented by the amino acid sequence of the following formula (1):
W-L₁-Xₙ-Y ... (1)
[wherein W represents an amino acid sequence including one or more amino acid sequences of a part or the entirety of a pathogenic virus non-structural protein serving as an immunogen; L₁ represents a first linker sequence having 0 to 100 amino acids; X represents an amino acid sequence represented by SEQ ID NO: 1; Y represents an amino acid sequence of a cell introduction domain; and repetition number n of X is an integer of 1 to 3],
wherein the amino acid sequence of the cell introduction domain Y is represented by the following formula (2) :
Y₁-L₂-Y₂-Y₃ ··· (2)
[wherein Y₁ represents any one amino acid sequence selected from the group consisting of SEQ ID NOs: 2 to 5; Y₂ represents any one amino acid sequence selected from the group consisting of SEQ ID NOs: 6 to 9; L₂ represents a second linker sequence having 0 to 30 amino acids; Y₃ represents an amino acid sequence for modification; and either of Y₂ and Y₃ may be absent],
wherein the amino acid sequence represented by Xₙ, Y₁, or Y₂ included in the above formulas may include a modified amino acid sequence thereof obtained by deleting, substituting, or adding one or more amino acid residues from, in, or to the original amino acid sequence.

2. The composite protein according to claim 1, wherein, in the modified amino acid sequence obtained through deletion, substitution, or addition of one or more amino acid residues from, in, or to the amino acid sequence represented by Xₙ, Y₁, or Y₂ included in the above formulas, the number of modifications of amino acid residues in each amino acid sequence is ≤8n in the case of Xₙ; ≤30 in the case of Y₁; and ≤15 in the case of Y₂.

3. A gene expression vector, to which a nucleic acid fragment encoding a composite protein as recited in claim 1 or 2 has been incorporated.

4. A transformant, which is formed through transformation with a nucleic acid fragment encoding a composite protein as recited in claim 1 or 2.

5. An associated product, which is formed through association of one or more composite proteins as recited in claim 1 or 2 in an aqueous liquid.

6. An associated product comprising a protein trimer and/or a protein hexamer formed of one or more composite proteins as recited in claim 1 or 2 as a protein monomer.

7. A component vaccine comprising, as an active ingredient, an associated product which has been formed through association of one or more composite proteins as recited in claim 1 or 2 in an aqueous liquid.

8. A component vaccine comprising, as an active ingredient, an associated product including a protein trimer and/or a protein hexamer formed of one or more composite proteins as recited in claim 1 or 2 as a protein monomer.

9. The component vaccine according to claim 7 or 8, which is to be administered subcutaneously, intradermally, percutaneously, mucosally, or intramuscularly.
